# EUROPEAN PATENT SPECIFICATION

(11) **EP 1 290 227 B1**
(45) Date of publication and mention of the grant of the patent: **12.08.2009**
(21) Application number: 01942050.4
(22) Date of filing: 05.06.2001
(51) Int. Cl.: C12Q 1/68, G01N 33/50, G01N 33/53, G01N 33/564, A61K 38/17, A61K 48/00, A61P 31/10

(54) **COMPOSITIONS, KITS, AND METHODS FOR IDENTIFICATION AND MODULATION OF TYPE I DIABETES**
ZUSAMMENSETZUNGEN, TESTSÄTZE UND METHODEN ZUR IDENTIFIZIERUNG UND MODULATION VON TYP I DIABETES
COMPOSITIONS, TROUSSES ET PROCEDES D'IDENTIFICATION ET DE MODULATION DU DIABETE DE TYPE I

(30) Priority: 05.06.2000 US 209703 P
(43) Date of publication of application: 12.03.2003
(73) Proprietor: Genetics Institute, LLC, Cambridge, MA 02140 (US); THE GENERAL HOSPITAL CORPORATION, Charlestown, MA 02129 (US)
(72) Inventor: BYRNE, Michael, C., Brookline, MA 02445 (US); HILL, Andrew, A., Cambridge, MA 02140 (US); WILSON, S., Brian, Lexington, MA 02421 (US)
(74) Representative: TBK-Patent
(86) International application number: PCT/US2001/018418
(87) International publication number: WO 2001/094636

(56) References cited:
- WO-A-93/19174
- WO-A-99/34209
- WILSON B ET AL: "Extreme Th1 bias of invariant Valpha 24JalphaQT cells in type 1 diabetes" NATURE, vol. 391, 8 January 1998 (1998-01-08), pages 177-81, XP002182875
- FALCONE M ET AL: "A defect in Interleukin 12-duced activation and interferon gamma secretion of peripheral natural killet T cells in nonobese diabetic mice suggests new pathogenic mechanisms for insulin-dependent diabetes" JOURNAL OF EXPERIMENTAL MEDICINE, vol. 190, no. 7, 4 October 1999 (1999-10-04), pages 963-972, XP002182876
- KIRSTEN J ET AL: "Alpha/beta-T cell receptor (TCR)+CD4-CD8- (NKT) thymocytes prevent insulin-dependent diabetes mellitus in nonobese diabetic (NOD)/Lt mice by the influence of interleukin (IL)-4 and/or IL-10" JOURNAL OF EXPERIMENTAL MEDICINE, vol. 187, no. 7, 6 April 1998 (1998-04-06), pages 1047-1056, XP002182877
- LEHUEN A ET AL: "Overexpressipon of natural killer T cells protects Valpha14-Jalpha281 transgenic nonobese diabetic mice against diabetes" JOURNAL OF EXPERIMENTAL MEDICINE, vol. 188, no. 10, 16 November 1998 (1998-11-16), pages 1831-39, XP002182878
- DERISI J ET AL: "USE OF A CDNA MICROARRAY TO ANALYSE GENE EXPRESSION PATTERNS IN HUMAN CANCER" NATURE GENETICS, NEW YORK, NY, US, vol. 11, 11 December 1996 (1996-12-11), pages 457-460, XP000971491 ISSN: 1061-4036

## Description

This application claims priority to U.S. Provisional Application No.: 60/209,703 filed on June 5,2000.

Diabetes mellitus is a syndrome with interrelated metabolic, vascular, and neuropathic components. The metabolic component, generally characterized by hyperglycemia, comprises alterations in carbohydrate, fat and protein metabolism caused by absent or markedly reduced insulin secretion and/or ineffective insulin action. The vascular component includes abnormalities in the blood vessels leading to cardiovascular, retinal and renal complications. Abnormalities in the peripheral and autonomic nervous systems comprise a third component of the diabetic syndrome.

There are two types of diabetes mellitus: type I and type II. Type I diabetes is also termed "insulin-dependent" diabetes, due to the fact that subjects afflicted with this disorder cannot synthesize their own insulin, and therefore must periodically inject insulin into their systems. Type II diabetic-afflicted subjects, on the other hand, are able to synthesize insulin, but this insulin is either insufficient for the needs of the subject, or is not effectively used by the subject. Type II diabetes ('non-insulin-dependent' diabetes) is typically controlled by oral medication.

Like many autoimmune diseases, Type I diabetes mellitus (IDDM) is a disorder with a highly complex etiology, which is thought to involve environmental "triggers" interacting with a polygenic genetic susceptibility. The earliest identification of a genetic locus conferring IDDM susceptibility occurred nearly thirty years ago, when the association of certain alleles of the major histocompatibility locus (MHC) with diabetes was discovered (Eisenbarth (1986) N. Engl. J. Med. 214(21):1360-1368; Wicker et al. (1995) Annu. Rev. Immunol. 13: 179-200; and Todd et al. (1987) Nature 329: 599-604). Subsequent epidemiological studies have conclusively demonstrated that the risk of developing diabetes is strongly correlated with the inheritance of certain MHC Class II alleles. While the MHC locus is the most significant genetic risk factor for diabetes, it accounts for less than 50% of this genetic risk, clearly indicating that non-MHC loci interspersed around the genome also make critical contributions to disease susceptibility and severity (Davies et al. (1994) Nature 371: 130-136).

Comprehensive genome-wide scans have to date located 18 different susceptibility loci for IDDM (Becker (1999) Diabetes 48(7): 1353-1358). These putative susceptibility loci must be evaluated with caution, as many of them possess statistical significance values less than the common standard (logarithm of odds [LOD]≥ 3) and only a portion of them have been replicated. Intriguingly, 15 out of the 18 candidate loci overlap with previously established susceptibility loci for other autoimmune diseases such as systemic lupus erythematosis (SLE), multiple sclerosis (MS), rheumatoid arthritis, ankylosing spondylitis and coeliac disease (Becker (1999) Diabetes 48(7): 1353-1358). While not definitive, this result could suggest that the candidate susceptibility loci might contain genes that play a central role in normal immune function and regulation. However, identification of the exact gene(s) in the putative susceptibility loci responsible for this immune regulation has proven far more difficult than identification of the loci themselves. Each of these loci can span enormous genetic distances of up to 10-30 cM in size and contain hundreds if not thousands of genes (Becker (1999) Diabetes 48(7): 1353-1358), many of which are of undefined function, and which may act combinatorially.

In a complementary approach to the identification of the underlying molecular basis of type I diabetes to that of genome-wide scans, the involvement of different immune cells in the disease has also been investigated. Invariant CD161⁺ V214Jα281 T cells (NKT cells) are known to be important in the regulation of T helper cell (Th) Th1/Th2 bias (Bendelac et al. (1997) Annu. Rev. Immunol. 15: 535-562), and NKT cells have been shown to be present in diminished numbers and to further decrease in frequency before the onset of disease in several murine models of autoimmunity (Takeda and Dennert (1993) J. Exp. Med. 177: 155-164; Mieza et al. (1996) J. Immunol. 156: 4035-4040; and Baxter et al. (1997) Diabetes 46: 572-582). When this population of cells was transferred from either nonobese diabetic or nonobese diabetic/Vα14Jα281-transgenic donors to prediabetic animals, the recipients were protected from diabetes (Baxter et al. (1997) Diabetes 46: 572-582; Lehuen et al. (1998) J. Exp. Med. 188:1831-1839). This transfer of protection was significantly inhibited by the coadministration of anti-IL-4 antibodies (Hammond et al. (1998) J. Exp. Med. 187: 1047-1056).

Humans have a homologous invariant *(i.e.,* with no N region additions) CD161⁺ Vα24JαQ T cell population whose restriction element, like that for the murine CD161⁺ Vα14Jα281 T cells, is the nonpolymorphic class Ib molecule CD1d (Exley et al. (1997) J. Exp. Med. 186(1):109-20). It has been shown that in five sets of monozygotic twins and triplets discordant for type 1 diabetes, invariant Vα24JαQ T cells were present at significantly higher frequencies in the nondiabetic siblings (Wilson et al. (1998) Nature 391(6663):177-81). Moreover, Vα24JαQ T cell clones from the nondiabetic siblings secreted both IL-4 and IFN-γ, whereas those derived from the diabetic siblings had an extreme impairment in the ability to secrete IL-4.

WO-A-93/19174 discloses a human T-cell differentiation marker called "HT6", an antibody directed against this marker, as well as a process for detecting the expression of this marker. HT6 is a protein expressed by human T-lymphocytes, which in particulars offers the possibility to detect Ti/CD3+, CD4+ and/or CD45RA- cells.

WILSON B ET AL: "Extreme Th1 bias of invariant Valpha 24JalphaQT cells in type 1 diabetes" NATURE, vol. 391, 8 January 1998 (1998-01-08), pages 177-81, and WO-A-99/34209 are directed to a decreased level of IL-4 secretion of (activated) diabetic NKT-cells compared to (activated) nondiabetic cells, which is correlated with the possible development of type I diabetes.

In one embodiment, the invention provides a method of assessing whether a sunject is afflicted with type I diabetes as defined in claim 1. In a preferred embodiment, the marker corresponds to a transcribed polynucleotide or portion thereof, where the polynucleotide includes the marker. In a particularly preferred embodiment, the level of expression of the marker in the sample differs from the normal level of expression of the marker in a subject not afflicted with type I diabetes by a factor of at least two, and in an even more preferred embodiment, the expression levels differ by a factor of at least five.

The sample includes NKT cells obtained from the subject. In another preferred embodiment, the level of expression of the marker in the sample is assessed by detecting the presence in the sample of a protein corresponding to the marker. In a particularly preferred embodiment, the presence of the protein is detected using a reagent which specifically binds with the protein. In an even more preferred embodiment, the reagent is selected from the group of reagents including an antibody, an antibody derivative, and an antibody fragment. In another preferred embodiment, the level of expression of the marker in the sample is assessed by detecting the presence in the sample of a transcribed polynucleotide or portion thereof, where the transcribed polynucleotide includes the marker. In a particularly preferred embodiment, the transcribed polynucleotide is an mRNA or a cDNA. In another particularly preferred embodiment, the step of detecting further comprises amplifying the transcribed polynucleotide.

In yet another preferred embodiment, the level of expression of the marker in the sample is assessed by detecting the presence in the sample of a transcribed polynucleotide which anneals with the marker or anneals with a portion of a polynucleotide under stringent hybridization conditions, where the polynucleotide includes the marker. In another preferred embodiment, the level of expression in the sample of each of a plurality of markers independently selected from the markers listed in Tables 1 and 4 is compared with the normal level of expression of each of the plurality of markers in samples of the same type obtained form control subjects not afflicted with type I diabetes, where the level of expression of more than one of the markers is significantly altered, relative to the corresponding normal levels of expression of the markers, is an indication that the subject is afflicted with type I diabetes. In a particularly preferred embodiment, the plurality includes two or more of the markers. In a still more preferred embodiment, the plurality includes at least five of the markers set forth in Tables 1 and 4.

In another embodiment, the invention provides a method for monitoring the progression of type I diabetes as defined in claim 18. In a preferred embodiment, the marker is selected from the group including the markers listed in Tables 1 and 4 and combinations thereof. In another preferred embodiment, the marker corresponds to a transcribed polynucleotide or portion thereof, where the polynucleotide includes the marker. The sample includes NKT cells obtained from the subject. In a particularly preferred embodiment, the NKT cells are collected from pancreatic or blood tissue.

In another embodiment, the invention provides a method of assessing the efficacy of a test compound for inhibiting type I diabetes as defined in claim 23. In a preferred embodiment, the first and second samples are portions of a single sample obtained from the subject In another preferred embodiment, the first and second samples are portions of pooled samples obtained from the subject.

In another embodiment, the invention provides a method of assessing the efficacy of a therapy for inhibiting type I diabetes as defined in claim 22.

In another embodiment, the invention provides a method of selecting a composition for inhibiting type I diabetes as defined in claim 26.

In another embodiment, the invention provides a use of a kit for conducting the methods of the invention as defined in claim 28.

In another embodiment, the invention provides a method of assessing the potential of a test compound to trigger type I diabetes as defined in claim 27.

Other features and advantages of the invention will be apparent from the following detailed description and claims.

### Brief Description of the Drawings

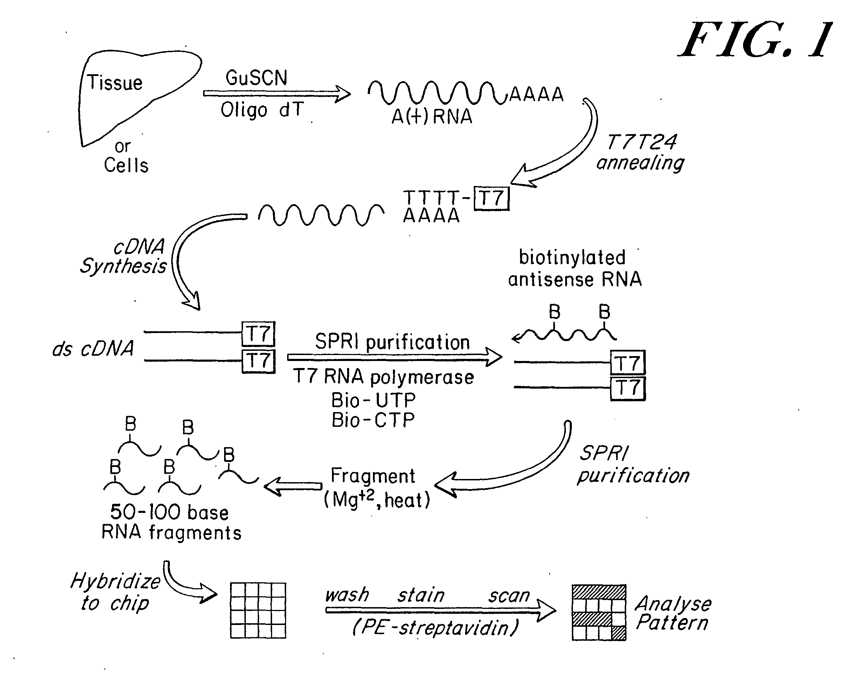

*Figure 1* is a graphical representation of the methodology used in the invention to determine the relative expression of marker genes in diabetic and nondiabetic cells.

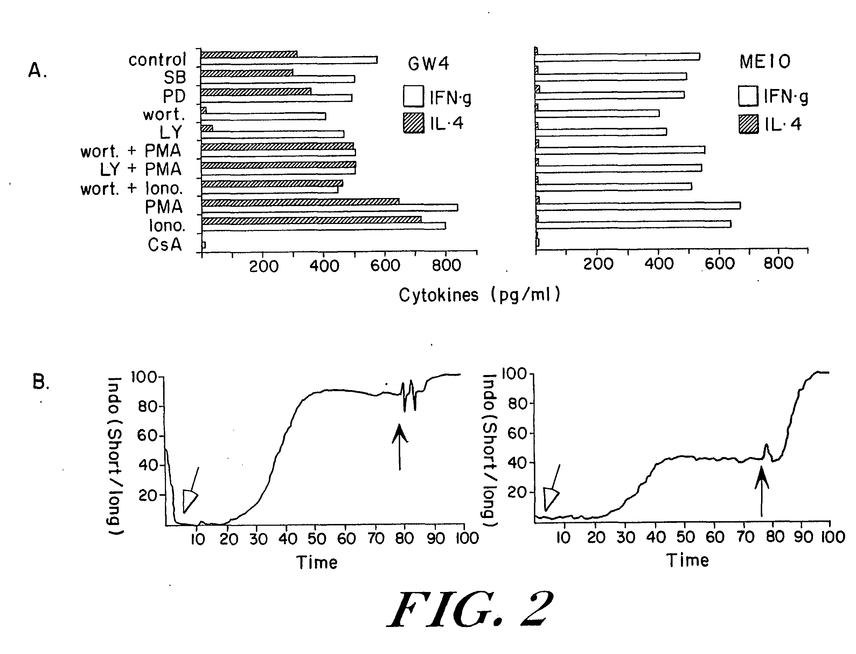

*Figure* 2 depicts the discordant expression of PI3-kinase-regulated events in IL-4⁺ and IL-4 null Vα24JαQ T cell (NKT) clones. Figure 2A shows bar graphs indicating the levels of IFN-γ and IL-4 secretion, as measured by ELISA assay, from Vα24JαQ T cell clones GW4 (nondiabetic) and ME10 (diabetic cells) activated with plate-bound anti-CD3 or an Ig control, in the presence or absence of various signal transduction pathway inhibitors or stimulators. The inhibitors used included wortmannin ('wort', 10 mM), LY294002 ('LY', 10 µM), PD98059 ('PD', 50 µM), S8203580 ('S8', 50 µM). The stimulators used were the phorbol ester PMA (1 ng/ml), ionomycin ('iono', 1 ng/ml), and cyclosporin A ('CsA', 5 ng/ml). Data points were collected in triplicate, and the data shown is representative of four independent experiments. Figure 2B depicts a graph of the fluorescence detected over time by flow cytometry (using a Cytomation MoFlo instrument) of the indo-1 labeled (10 µM) and anti-CD3 (10 µg/ml) stimulated Vα24JαQ T cell clones CW4 (IL-4⁺) and ME10 (IL-4-null). At the end of the experiment, ionomycin was added to a final concentration of 1 µg/ml to determine maximal flux. The ratio of Indo-1 fluorescence at 410/490 nm (410: Ca2⁺ bound; 490: Ca2⁺ free) after stimulation in a representative pair of clones is pictured.

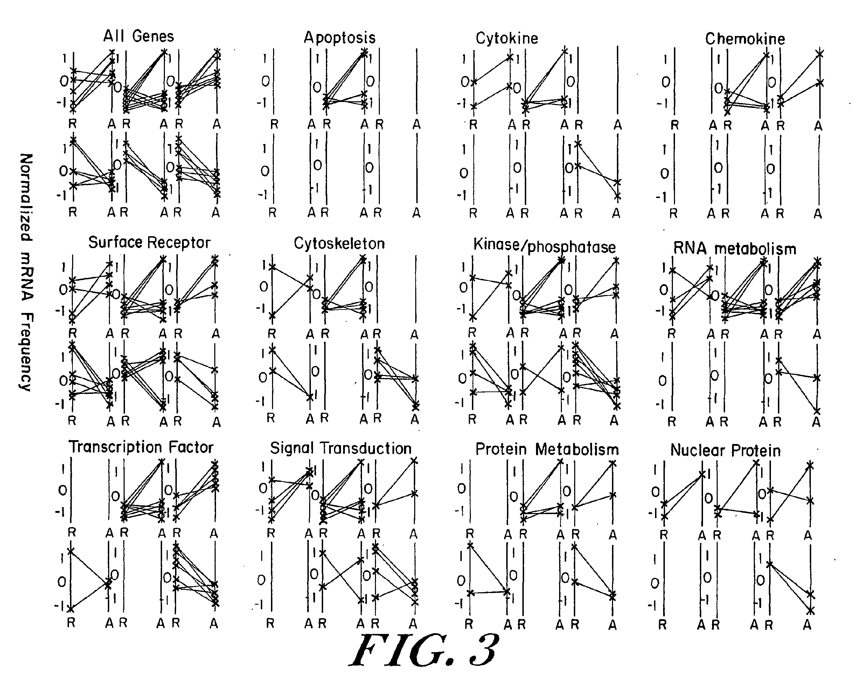

*Figure* 3 is a graphical representation of genes differentially expressed in NKT cell clones derived form a diabetic/non-diabetic twin pair. Clones ME10 (diabetic) and GW4 (non-diabetic) were treated with control IgG (designated R = Resting) or anti-CD3 (designated A = Activated) for four hours, after which RNA was isolated and analyzed on genechips monitoring the expression of 6800 human genes from the Unigene collection. Genes whose expression was modulated at least 2-fold in either clone were chosen for clustering analysis using the Self-Organizing Map algorithm (Tamayo *et al.* (1999)). This method was used to cluster genes into six distinct groups, based on differential expression patterns between ME10 and GW4, independent of expression magnitude. The first group displays the six patterns represented when all genes meeting the 2-fold change criterion are used. The other groupings reveal the differential expression patterns of selected gene functional classes.

The invention relates, in part, to newly discovered correlations between the expression of selected markers in NKT cells and the presence of type I diabetes in a subject. The relative levels of expression of these markers, both alone and in combination, have been found to be indicative of a predisposition in the subject to type I diabetes and/or diagnostic of the presence or potential presence of type I diabetes in a subject. The invention provides panels of markers, methods for detecting the presence or absence of type I diabetes in a sample or subject, and methods of predicting the incidence of type I diabetes in a sample or subject.

The present invention is based, at least in part, on the identification of a number of genetic markers, set forth in Tables 1 and 4, which are differentially expressed in activated NKT cells from a diabetic subjects relative to a nondiabetic subject. A panel of 6800 known genes was screened for expression in activated diabetic NKT cells versus activated nondiabetic NKT cells taken from identical twins discordant for type I diabetes (see Example 2). Those genes with at least two-fold differences between the diseased and normal activated cells are identified in Tables 1 and 4.

Six different expression patterns were observed in activated diabetic versus nondiabetic NKT cells. Table 1 (representative of row 1, column 1 in each of the clusters set forth in Figure 3) lists each of the genes which were observed to be increased in expression in activated diabetic NKT cells and unchanged or increasing to a lesser extent in expression in activated nondiabetic NKT cells, relative to appropriate resting control cells. Table 2 (representative of row 1, column 2 in each of the clusters set forth in Figure 3; outside the scope of the invention) lists each of the genes which were observed to be unchanged in expression in activated diabetic NKT cells relative to control resting cells, but which are increased in expression in activated nondiabetic NKT cells relative to resting control cells. Table 3 (representative of row 1, column 3 in each of the clusters set forth in Figure 3 (outside the scope of the invention)) lists each of the genes which were observed to be increased in expression in both activated diabetic and nondiabetic NKT cells relative to appropriate resting control cells. Table 4 (representative of row 2, column 1 in each of the clusters set forth in Figure 3) lists those genes which were observed to be decreased in expression in activated nondiabetic NKT cells relative to resting control cells, but which were unchanged or decreasing to a lesser extent in expression in activated diabetic NKT cells relative to resting control cells. Table 5 (representative of row 2, column 2 sin each of the clusters set forth in Figure 3; outside the scope of the invention) lists those genes which were observed to be increased in expression in activated nondiabetic NKT cells relative to resting control cells, but which were decreased in expression in activated diabetic NKT cells relative to resting control cells. Table 6 (representative of row 2, column 3 in each of the clusters set forth in Figure 3; outside the scope of the invention) lists those genes which were observed to be decreased in expression in activated diabetic NKT cells relative to resting control cells, but which were unchanged or decreasing to a lesser extent in expression in nondiabetic NKT cells relative to resting control cells.

NKT, CD4, and CD8 T cell clones were generated and stimulated with anti-CD3 for 2,4,8,24, or 48 hours. Genes which were identified in a query requiring at least a three-fold increase in mRNA levels at least one time point in NKT cell samples are set forth in Table 9 (outside the scope of the invention). Genes which were identified in a query requiring at least a three-fold increase in mRNA levels in at least one time point for all three replications of the experiment in CD4 cell samples are set forth in Table 10 (outside the scope of the invention). Genes which were identified in a query requiring at least a three-fold increase in mRNA levels in at least one time point for all three replications of the experiment in CD8 cell samples are set forth in Table 11 (outside the scope of the invention).

NKT, CD4, and CD8 T cell clones were generated. Genes which were identified in a query requiring at least a three-fold change in mRNA levels for all three replications of the experiment in resting CD4 cell samples relative to resting NKT cell samples are set forth in Table 12 (outside the scope of the invention). Genes which were identified in a query requiring at least a three-fold change in mRNA levels for all three replications of the experiment in resting CD8 cell samples relative to resting NKT cell samples are set forth in Table 13 (outside the scope of the invention).

Several genes were identified which were differentially regulated in resting (*e*.*g*., unactivated) diabetic versus nondiabetic NKT cells. These genes are set forth in . Table 8.

There are several genes known in the art to be implicated in type I diabetes, set forth in Table 7. These genes are not meant to be used singly in the methods, compositions, and kits of the invention, but may be used in combination with the markers of the invention set forth in Tables 1 and 4.

Accordingly, the present invention pertains to the use of the genes set forth in Tables 1 and 4 (*e*.*g*., the DNA or cDNA), the corresponding mRNA transcripts, and the encoded polypeptides as markers for the presence or risk of development of type I diabetes. These markers are further useful to correlate the extent and/or severity of disease. Panels of the markers can be conveniently arrayed for use in kits or on solid supports. The markers can also be useful in assessing the efficacy of a treatment for type I diabetes.

In one-aspect, markers whose quantity or activity is correlated with the presence of type I diabetes are provided. The markers may be nucleic acid molecules (*e*.*g*., DNA, cDNA, or RNA) or polypeptides. These markers are either increased or decreased in quantity or activity in diabetic NKT cells as compared to nondiabetic NKT cells. For example, the IFN-γ gene (outside the scope of the invention) (accession number J00219) is increased in expression level in activated nondiabetic NKT cells but not in activated diabetic NKT cells (Table 2), while the lymphotoxin-beta gene (designated 'LT-β') (accession number U89922) is increased in expression in activated diabetic NKT cells but not in activated nondiabetic NKT cells (Table 1). Both the presence of increased or decreased mRNA for these genes (and for other genes set forth in Tables 1 and 4), and also increased or decreased levels of the protein products of these genes (and other genes set forth in Tables 1 and 4) serve as markers of type I diabetes. Preferably, increased and decreased levels of the markers are increases and decreases of a magnitude that is statistically significant as compared to appropriate control samples (*e*.*g*., samples not affected with type I diabetes or an NKT-associated condition). In particularly preferred embodiments, the marker is increased or decreased relative to control samples by at least 2-, 3-, 4-, 5-, 6-, 7-, 8-, 9-, or 10-fold or more. Similarly, one skilled in the art will be cognizant of the fact that a preferred detection methodology is one in which the resulting detection values are above the minimum detection limit of the methodology.

Measurement of the relative amount of an RNA or protein marker of the invention may be by any method known in the art (see, *e*.*g*., Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989; and Current Protocols in Molecular Biology, eds. Ausubel et al. John Wiley & Sons: 1992). Typical methodologies for RNA detection include RNA extraction from a cell or tissue sample, followed by hybridization of a labeled probe (*e*.*g*., a complementary nucleic acid molecule) specific for the target RNA to the extracted RNA, and detection of the probe (*e*.*g*., Northern blotting). Typical methodologies for protein detection include protein extraction from a cell or tissue sample, followed by hybridization of a labeled probe (*e*.*g*., an antibody) specific for the target protein to the protein sample, and detection of the probe. The label group can be a radioisotope, a fluorescent compound, an enzyme, or an enzyme co-factor. Detection of specific protein and nucleic acid molecules may also be assessed by gel electrophoresis, column chromatography, direct sequencing, or quantitative PCR (in the case of nucleic acid molecules) among many other techniques well known to those skilled in the art.

In certain embodiments, the genes themselves (*e.g.,* the DNA or cDNA) may serve as markers for type I diabetes. For example, the absence of nucleic acids corresponding to a gene (*e*.*g*., a gene from Table 1), such as by deletion of all or part of the gene, may be correlated with disease. Similarly, an increases of nucleic acid corresponding to a gene such as by duplication of the gene, may also be correlated with disease.

Detection of the presence or number of copies of all or a part of a marker gene may be performed using any method known in the art. Typically, it is convenient to assess the presence and/or quantity of a DNA or cDNA by Southern analysis, in which total DNA from a cell or tissue sample is extracted, is hybridized with a labeled probe (*e*.*g*., a complementary DNA molecule), and the probe is detected. The label group can be a radioisotope, a fluorescent compound, an enzyme, or an enzyme co-factor. Other useful methods of DNA detection and/or quantification include direct sequencing, gel electrophoresis, column chromatography, and quantitative PCR, as is known by one skilled in the art.

Nucleic acid and protein molecules which are structurally different from the molecules described above (*e*.*g*., which have a slightly altered nucleic acid or amino acid sequence), but which have the same properties as the molecules above (*e*.*g*., encoded amino acid sequence, or which are changed only in nonessential amino acid residues) are also described. Such molecules include allelic variants, and are described in greater detail in subsection I.
In another aspect, markers whose quantity or activity is correlated with the severity of type I diabetes (see *e*.*g*., Example 3) are described. These markers are either increased or decreased in quantity or activity in diabetic NKT cells in a fashion that is either positively or negatively correlated with the degree of severity of the type I diabetes. In yet another aspect, markers whose quantity or activity is correlated with a risk in a subject for developing type I diabetes are described. These markers are either increased or decreased in activity or quantity in direct correlation to the likelihood of the development of type I diabetes in a subject.

Each marker may be considered individually, although combinations of two or more markers for use in the methods and compositions of the invention to increase the confidence of the analysis are also provided. In another aspect, panels of the markers are also provided. Panels of the markers of the invention are set forth in Tables 1 and 4. It will be apparent to one skilled in the art that the methods of the invention may be practiced with any one of the panels set forth in Tables 1 and 4, any portion or combination thereof.

It will also be appreciated by one skilled in the art that the panels of markers may conveniently be provided on solid supports. For example, polynucleotides, such as oligonucleotides or cDNA, may be coupled to an array (*e*.*g*., a GeneChip array for hybridization analysis), to a resin (*e*.*g*., a resin which can be packed into a column for column chromatography), or a matrix (*e*.*g*., a nitrocellulose matrix for northern blot analysis). The immobilization of molecules complementary to the marker(s), either covalently or noncovalently, permits a discrete analysis of the presence or activity of each marker in a sample. In an array, for example, polynucleotides, complementary to each member of a panel of markers may individually be attached to different, known locations on the array. The array may be hybridized with, for example, polynucleotides extracted from a blood sample from a subject. The hybridization of polynucleotides from the sample with the array at any location on the array can be detected, and thus the presence or quantity of the marker in the sample can be ascertained. In a preferred embodiment, a "GeneChip" array is employed (Affymetrix). Similarly, Western analyses may be performed on immobilized antibodies specific for different polypeptide markers hybridized to a protein sample from a subject.

It will also be apparent to one skilled in the art that the entire marker protein or nucleic acid molecule need not be conjugated to the support; a portion of the marker of sufficient length for detection purposes (*e*.*g*., for hybridization), for example, a portion of the marker which is 7,10,15,20,25,30,35,40,45,50,55,60,65,70,75,100 or more nucleotides or amino acids in length may be sufficient for detection purposes.

The nucleic acid and protein markers disclosed may be isolated from any tissue or cell of a subject. The cells are NKT cells. However, it will be apparent to one skilled in the art that other tissue samples, including bodily fluids (e.g., urine, bile, serum, lymph, saliva, mucus and pus), among other tissue samples, may also serve as sources from which the markers may be isolated, or in which the presence, activity, and/or quantity of the markers may be assessed. The tissue samples containing one or more of the markers themselves may be useful in the methods disclosed, and one skilled in the art will be cognizant of the methods by which such samples may be conveniently obtained, stored, and/or preserved.

Several markers were known prior to the invention to be associated with diabetes. These markers are set forth in Table 7. These markers are not included with the markers of the invention. However, these markers may conveniently be used in combination with the markers of the invention in the methods, panels, and use of the invention.

The invention also provides the use of kits for assessing the presence of diabetic NKT cells in a sample (*e.g.*, a sample from a subject at risk for type I diabetes), the kit comprising an antibody, wherein the antibody specifically binds with a protein corresponding to a marker selected from the group consisting of the markers listed in Tables 1 and 4.

The invention further provides the use of kits for assessing the presence of type I diabetic NKT cells in a sample from a subject *(e.g.,* a subject at risk for type I diabetes), the kit comprising a nucleic acid probe wherein the probe specifically binds with a transcribed polynucleotide corresponding to a marker selected from the group consisting of the markers listed in Tables 1 and 4.

The invention further provides the use of kits for assessing the suitability of each of a plurality of compounds for inhibiting type I diabetes in a subject. Such kits include a plurality of compounds to be tested, and a reagent for assessing expression of a marker selected from the group consisting of one or more of the markers set forth in Tables 1 and 4.

Modifications to the above-described compositions and methods of the invention, according to standard techniques, will be readily apparent to one skilled in the art.

To facilitate an understanding of the present invention, a number of terms and phrases are defined below:

As used herein, the terms "polynucleotide" and "oligonucleotide" are used interchangeably, and include polymeric forms of nucleotide of any length, either deoxyribonucleotides or ribonucleotides, or analogs thereof. Polynucleotides may have any three-dimensional structure, and may perform any function, known or unknown, The following are non-limiting examples of polynucleotides: a gene or gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers. A polynucleotide may comprise modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The term also includes both double- and single-stranded molecules. Unless otherwise specified or required, any embodiment of this invention that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (IJ) for guanine when the polynucleotide is RNA. This, the term "polynucleotide sequence" is the alphabetical representation of a polynucleotide molecule. This alphabetical representation can be inputted into databases in a computer having a central processing unit and used for bioinformatics applications such as functional genomics and homology searching.

A "gene" includes a polynucleotide containing at least one open reading frame that is capable of encoding a particular polypeptide or protein after being transcribed and translated. Any of the polynucleotide sequences described herein may be used to identify larger fragments or full-length coding sequences of the gene with which they are associated. Methods of isolating larger fragment sequences are known to those of skill in the art, some of which are described herein.

A "gene product" includes an amino acid (e.g., peptide or polypeptide) generated when a gene is transcribed and translated.

As used herein, a "polynucleotide corresponds to" another (a first) polynucleotide if it is related to the first polynucleotide by any of the following relationships:
1) The second polynucleotide comprises the first polynucleotide and the second polynucleotide encodes a gene product.
2) The second polynucleotide is 5' or 3' to the first polynucleotide in cDNA, RNA, genomic DNA, or fragments of any of these polynucleotides. For example, a second polynucleotide may be a fragment of a gene that includes the first and second polynucleotides. The first and second polynucleotides are related in that they are components of the gene coding for a gene product, such as a protein or antibody. However, it is not necessary that the second polynucleotide comprises or overlaps with the first polynucleotide to be encompassed within the definition of "corresponding to" as used herein. For example, the first polynucleotide may be a fragment of a 3' untranslated region of the second polynucleotide. The first and second polynucleotide may be fragments of a gene coding for a gene product. The second polynucleotide may be an exon of the gene while the first polynucleotide may be an intron of the gene.
3) The second polynucleotide is the complement of the first polynucleotide.

A "probe" when used in the context of polynucleotide manipulation includes an oligonucleotide that is provided as a reagent to detect a target present in a sample of interest by hybridizing with the target. Usually, a probe will comprise a label or a means by which a label can be attached, either before or subsequent to the hybridization reaction. Suitable labels include, but are not limited to radioisotopes, fluorochromes, chemiluminescent compounds, dyes, and proteins, including enzymes.

A "primer" includes a short polynucleotide, generally with a free 3'-OH group that binds to a target or "template" present in a sample of interest by hybridizing with the target, and thereafter promoting polymerization of a polynucleotide complementary to the target. A "polymerase chain reaction" ("PCR") is a reaction in which replicate copies are made of a target polynucleotide using a "pair of primers" or "set of primers" consisting of "upstream" and a "downstream" primer, and a catalyst of polymerization, such as a DNA polymerase, and typically a thermally-stable polymerase enzyme. Methods for PCR are well known in the art, and are taught, for example, in MacPherson et al. , IRL Press at Oxford University Press (1991)). All processes of producing replicate copies of a polynucleotide, such as PCR or gene cloning, are collectively referred to herein as "replication". A primer can also be used as a probe in hybridization reactions, such as Southern or Northern blot analyses (see, *e.g*., Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989).

The term "cDNAs" includes complementary DNA, that is mRNA molecules present in a cell or organism made into cDNA with an enzyme such as reverse transcriptase. A "cDNA library" includes a collection of mRNA molecules present in a cell or organism, converted into cDNA molecules with the enzyme reverse transcriptase, then inserted into "vectors" (other DNA molecules that can continue to replicate after addition of foreign DNA). Exemplary vectors for libraries include bacteriophage, viruses that infect bacteria (e.g., lambda phage). The library can then be probed for the specific cDNA (and thus mRNA) of interest.

A "gene delivery vehicle" includes a molecule that is capable of inserting one or more polynucleotides into a host cell. Examples of gene delivery vehicles are liposomes, biocompatible polymers, including natural polymers and synthetic polymers; lipoproteins; polypeptides; polysaccharides; lipopolysaccharides; artificial viral envelopes; metal particles; and bacteria, viruses and viral vectors, such as baculovirus, adenovirus, and retrovirus, bacteriophage, cosmid, plasmid, fungal vector and other recombination vehicles typically used in the art which have been described for replication and/or expression in a variety of eukaryotic and prokaryotic hosts. The gene delivery vehicles may be used for replication of the inserted polynucleotide, gene therapy as well as for simply polypeptide and protein expression.

A "vector" includes a self-replicating nucleic acid molecule that transfers an inserted polynucleotide into and/or between host cells. The term is intended to include vectors that function primarily for insertion of a nucleic acid molecule into a cell, replication vectors that function primarily for the replication of nucleic acid and expression vectors that function for transcription and/or translation of the DNA or RNA. Also intended are vectors that provide more than one of the above function.

A "host cell" is intended to include any individual cell or cell culture which can be or has been a recipient for vectors or for the incorporation of exogenous nucleic acid molecules, polynucleotides and/or proteins. It also is intended to include progeny of a single cell. The progeny may not necessarily be completely identical (in morphology or in genomic or total DNA complement) to the original parent cell due to natural, accidental, or deliberate mutation. The cells may be prokaryotic or eukaryotic, and include but are not limited to bacterial cells, yeast cells, insect cells, animal cells, and mammalian cells, e.g., murine, rat, simian or human cells.

The term "genetically modified" includes a cell containing and/or expressing a foreign gene or nucleic acid sequence which in turn modifies the genotype or phenotype of the cell or its progeny. This term includes any addition, deletion, or disruption to a cell's endogenous nucleotides.

As used herein, "expression" includes the process by which polynucleotides are transcribed into mRNA and translated into peptides, polypeptides, or proteins. If the polynucleotide is derived from genomic DNA, expression may include splicing of the mRNA, if an appropriate eukaryotic host is selected. Regulatory elements required for expression include promoter sequences to bind RNA polymerase and transcription initiation sequences for ribosome binding. For example, a bacterial expression vector includes a promoter such as the lac promoter and for transcription initiation the Shine-Dalgarno sequence and the start codon AUG (Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989). Similarly, a eukaryotic expression vector includes a heterologous or homologous promoter for RNA polymerase II, a downstream polyadenylation signal, the start codon AUG, and a termination codon for detachment of the ribosome. Such vectors can be obtained commercially or assembled by the sequences described in methods well known in the art, for example, the methods described below for constructing vectors in general.

"Differentially expressed", as applied to a gene, includes the differential production of mRNA transcribed from a gene or a protein product encoded by the gene. A differentially expressed gene may be overexpressed or underexpressed as compared to the expression level of a normal or control cell. In one aspect, it includes a differential that is 2.5 times, preferably 5 times or preferably 10 times higher or lower than the expression level detected in a control sample. The term "differentially expressed" also includes nucleotide sequences in a cell or tissue which are expressed where silent in a control cell or not expressed where expressed in a control cell.

The term "polypeptide" includes a compound of two or more subunit amino acids, amino acid analogs, or peptidomimetics. The subunits may be linked by peptide bonds. In another embodiment, the subunit may be linked by other bonds, e.g., ester, ether, etc. As used herein the term "amino acid" includes either natural and/or unnatural or synthetic amino acids, including glycine and both the D or L optical isomers, and amino acid analogs and peptidomimetics. A peptide of three or more amino acids is commonly referred to as an oligopeptide. Peptide chains of greater than three or more amino acids are referred to as a polypeptide or a protein.

"Hybridization" includes a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. A hybridization reaction may constitute a step in a more extensive process, such as the initiation of a PCR reaction, or the enzymatic cleavage of a polynucleotide by a ribozyme.

Hybridization reactions can be performed under conditions of different "stringency". The stringency of a hybridization reaction includes the difficulty with which any two nucleic acid molecules will hybridize to one another. Under stringent conditions, nucleic acid molecules at least 60%, 65%, 70%, 75% identical to each other remain hybridized to each other, whereas molecules with low percent identity cannot remain hybridized. A preferred, non-limiting example of highly stringent hybridization conditions are hybridization in 6X sodium chloride/sodium citrate (SSC) at about 45°C, followed by one or more washes in 0.2 X SSC, 0.1% SDS at 50°C, preferably at 55°C, more preferably at 60°C, and even more preferably at 65°C.

When hybridization occurs in an antiparallel configuration between two single-stranded polynucleotides, the reaction is called "annealing" and those polynucleotides are described as "complementary". A double-stranded polynucleotide can be "complementary" or "homologous" to another polynucleotide, if hybridization can occur between one of the strands of the first polynucleotide and the second. "Complementarity" or "homology" (the degree that one polynucleotide is complementary with another) is quantifiable in terms of the proportion of bases in opposing strands that are expected to hydrogen bond with each other, according to generally accepted base-pairing rules.

An "antibody" includes an immunoglobulin molecule capable of binding an epitope present on an antigen. As used herein, the term encompasses not only intact immunoglobulin molecules such as monoclonal and polyclonal antibodies, but also anti-idotypic antibodies, mutants, fragments, fusion proteins, bi-specific antibodies, humanized proteins, and modifications of the immunoglobulin molecule that comprises an antigen recognition site of the required specificity.

As used herein, the term "type I diabetes" includes a noncontagious disorder wherein a subject is unable to manufacture insulin. Symptoms of type I diabetes include weight loss, irritability, frequent urination, excessive thirst, extreme hunger, weakness or fatigue, and nausea or vomiting.

As used herein, the term "NKT cell" includes cells which are identified by the expression of both natural killer (NK) cell markers and an invariant T cell-receptor. Such cells are CD161⁺, and are also known as Vα24JαQ T cells.

As used herein, the term "diabetic tissue" or "diabetic cell" or "type I diabetic tissue" or "type I diabetic cell" includes a tissue or cell from a subject afflicted with type I diabetes, where the tissue itself is involved in the symptomology of type I diabetes. An example of a type I diabetic tissue is pancreatic beta cells (insulin-producing cells) or blood. A "non-diabetic tissue" or "non-diabetic cell" includes a tissue or cell which either is from a subject not afflicted with type I diabetes, or which is from a subject afflicted with type I diabetes, but in which the tissue or cell itself is not involved in the symptomology of the disease, and/or is not affected by the presence of the disease. A "diabetic NKT cell", therefore, is an NKT cell taken from a diabetic subject. A "nondiabetic NKT cell" is an NKT cell taken from a nondiabetic subject.

As used herein, the term "marker" includes a polynucleotide or polypeptide ' molecule which is present or absent, or increased or decreased in quantity or activity in subjects afflicted with type I diabetes, or in cells involved in type I diabetes. The relative change in quantity or activity of the marker is correlated with the incidence or risk of incidence of type I diabetes.

As used herein, the term "panel of markers" includes a group of markers, the quantity or activity of each member of which is correlated with the incidence or risk of incidence of type I diabetes. In certain embodiments, a panel of markers may include only those markers which are either increased or decreased in quantity or activity in subjects afflicted with or cells involved in type I diabetes. In other embodiments, a panel of markers may include only those markers present in a specific tissue type which are correlated with the incidence or risk of incidence of type I diabetes.

Various aspects are described in further detail in the following subsections:

### I. Isolated Nucleic Acid Molecules

One aspect pertains to isolated nucleic acid molecules that either themselves are the genetic markers (*e.g*.. mRNA) of the invention, or which encode the , polypeptide markers of the invention, or fragments thereof. Another aspect pertains to isolated nucleic acid fragments sufficient for use as hybridization probes to identify the nucleic acid molecules encoding the markers disclosed in a sample, as well as nucleotide fragments for use as PCR primers for the amplification or mutation of the nucleic acid molecules which encode the markers disclosed. As used herein, the term "nucleic acid molecule" is intended to include DNA molecules (*e.g.,* cDNA or genomic DNA) and RNA molecules (*e.g*., mRNA) and analogs of the DNA or RNA generated using nucleotide analogs. - The nucleic acid molecules can be single-stranded or double-stranded, but preferably is double-stranded DNA).

The term "isolated nucleic acid molecule" includes nucleic acid molecules which are separated from other nucleic acid molecules which are present in the natural z source of the nucleic acid. For example, with regards to genomic DNA, the term "isolated" includes nucleic acid molecules which are separated from the chromosome with which the genomic DNA is naturally associated. Preferably, an "isolated" nucleic acid is free of sequences which naturally flank the nucleic acid (*i.e.,* sequences located at the 5' and 3' ends of the nucleic acid) in the genomic DNA of the organism from which the nucleic acid is derived. For example, in various embodiments, the isolated marker nucleic acid molecule, or nucleic acid molecule encoding a polypeptide marker, can contain less than about 5 kb, 4kb, 3kb, 2kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule disclosed, *e.g*., a nucleic acid molecule having the nucleotide sequence of one of the genes set forth in Tables 1-13, or a portion thereof, can be isolated using standard molecular biology techniques and the sequence information provided herein. Using all or portion of the nucleic acid sequence of one of the genes set forth in Tables 1-13 as a hybridization probe, a marker gene or a nucleic acid molecule encoding a polypeptide marker can be isolated using standard hybridization and cloning techniques (*e.g*., as described in Sambrook, J., Fritsh, E. F., and Maniatis, T. *Molecular Cloning: A Laboratory Manual 2nd, ed., Cold Spring Harbor Laboratory,* Cold Spring Harbor Laboratory Press, Cold Spring Harbor, NY, 1989).

A nucleic acid disclosed can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to marker nucleotide sequences, or nucleotide sequences encoding a marker disclosed can be prepared by standard synthetic techniques, *e.g*., using an automated DNA synthesizer.

In another preferred embodiment, an isolated nucleic acid molecule comprises a nucleic acid molecule which is a complement of the nucleotide sequence of a marker disclosed (*e.g*., a gene set forth in Tables 1-13), or a portion of any of these nucleotide sequences. A nucleic acid molecule which is complementary to such a nucleotide sequence is one which is sufficiently complementary to the nucleotide sequence such that it can hybridize to the nucleotide sequence, thereby forming a stable duplex.

The nucleic acid molecule moreover, can comprise only a portions of the nucleic acid sequence of a marker nucleic acid disclosed, or a gene encoding a marker polypeptide disclosed, for example, a fragment which can be used as a probe or primer. The probe/primer typically comprises substantially purified oligonucleotide. The oligonucleotide typically comprises a region of nucleotide sequence that hybridizes under stringent conditions to at least about 7 or 15, preferably about 20 or 25, more preferably about 50, 75,100,125, 150, 175, 200, 225, 250, 275, 300, 325, 350, 400 or more consecutive nucleotides of a marker nucleic acid, or a nucleic acid encoding a marker polypeptide disclosed.

Probes based on the nucleotide sequence of a marker gene or of a nucleic acid molecule encoding a marker polypeptide disclosed can be used to detect transcripts or genomic sequences corresponding to the marker gene(s) and/or marker polypeptide(s) disclosed. In preferred embodiments, the probe comprises a label group attached thereto, *e.g.*, the label group can be a radioisotope, a fluorescent compound, an enzyme, or an enzyme co-factor. Such probes can be used as a part of a diagnostic test kit for identifying cells or tissue which misexpress (*e.g.*, over- or under-express) a marker polypeptide disclosed, or which have greater or fewer copies of a marker gene disclosed. For example, a level of a marker polypeptide-encoding nucleic acid in a sample of cells from a subject may be detected, the amount of mRNA transcript of a gene encoding a marker polypeptide may be determined, or the presence of mutations or deletions of a marker gene disclosed may be assessed.

Further, nucleic acid molecules that differ from the nucleic acid sequences of the genes set forth in Tables 1 and 4, due to degeneracy of the genetic code and which thus encode the same proteins as those encoded by the genes shown in Tables 1 and 4, are described.

In addition to the nucleotide sequences of the genes set form in Tables 1 and 4, it will be appreciated by those skilled in the art that DNA sequence polymorphisms that lead to changes in the amino acid sequences of the proteins encoded by the genes set forth in Tables 1 and 4 may exist within a population (*e.g*., the human population). Such genetic polymorphism in the genes set forth in Tables 1 and 4 may exist among individuals within a population due to natural allelic variation. An allele is one of a group of genes which occur alternatively at a given genetic locus. In addition it will be appreciated that DNA polymorphisms that affect RNA expression levels can also exist that may affect the overall expression level of that gene (*e.g*., by affecting regulation or degradation). As used herein, the phrase "allelic variant" includes a nucleotide sequence which occurs at a given locus or to a polypeptide encoded by the nucleotide sequence. As used herein, the terms "gene" and "recombinant gene" refer to nucleic acid molecules which include an open reading frame encoding a marker polypeptide disclosed.

Nucleic acid molecules corresponding to natural allelic variants and homologues of the marker genes, or genes encoding the marker proteins disclosed can be isolated based on their homology to the genes set forth in Tables 1 and 4, using the cDNAs disclosed herein, or a portion thereof, as a hybridization probe according to standard hybridization techniques under stringent hybridization conditions. Nucleic acid molecules corresponding to natural allelic variants and homologues of the marker genes disclosed can further be isolated by mapping to the same chromosome or locus as the marker genes or genes encoding the marker proteins disclosed.

In another embodiment, an isolated nucleic acid molecule disclosed is at least 15, 20, 25, 30, 50, 100,150, 200, 250, 300, 350, 400, 450, 500, 550, 600, 650, 700, 750,800,850,900,950, 1000, 1100,1200, 1300, 1400, 1500, 1600, 1700,1800, 1900, 2000 or more nucleotides in length and hybridizes under stringent conditions to a nucleic acid molecule corresponding to a nucleotide sequence of a marker gene or gene encoding a marker protein disclosed. As used herein, the term "hybridizes under stringent conditions" is intended to describe conditions for hybridization and washing under which nucleotide sequences at least 60% homologous to each other typically remain hybridized to each other. Preferably, the conditions are such that sequences at least about 70%, more preferably at least about 80%, even more preferably at least about 85% or 90% homologous to each other typically remain hybridized to each other. Such stringent conditions are known to those skilled in the art and can be found in *Current Protocols in Molecular Biology,* John Wiley & Sons, N.Y. (1989), 6.3.1-6.3.6. A preferred, non-limiting example of stringent hybridization conditions are hybridization in 6X sodium chloride/sodium citrate (SSC) at about 45°C, followed by one or more washes in 0.2 X SSC, 0.1% SDS at 50°C, preferably at 55°C, more preferably at 60°C, and even more preferably at 65°C. Preferably, an isolated nucleic acid molecule that hybridizes under stringent conditions to the sequence of one of the genes set forth in Tables 1-13 corresponds to a naturally-occurring nucleic acid molecule. As used herein, a "naturally-occurring" nucleic acid molecule includes an RNA or DNA molecule having a nucleotide sequence that occurs in nature (*e.g*., encodes a natural protein).

In addition to naturally-occurring allelic variants of the marker gene and gene encoding a marker protein disclosed sequences that may exist in the population, the skilled artisan will further appreciate that changes can be introduced by mutation into the nucleotide sequences of the marker genes or genes encoding the marker proteins disclosed, thereby leading to changes in the amino acid sequence of the encoded proteins, without altering the functional activity of these proteins. For example, nucleotide substitution leading to amino acid substitutions at "non-essential" amino acid residues can be made. A "non-essential"'amino acid residue is a residue that can be altered from the wild type sequence of a protein without altering the biological activity, whereas an "essential" amino acid residue is required for biological activity. For example, amino acid residues that are conserved among allelic variants or homologs of a gene (e.g., among homologs of a gene from different species) are predicted to be particularly unamenable to alteration.

Accordingly, another aspect pertains to nucleic acid molecules encoding a marker protein disclosed that contain changes in amino acid residues that are not essential for activity. Such proteins differ in amino acid sequence from the marker proteins encoded by the genes set forth in Tables 1 and 4, yet retain biological activity. In one embodiment, the protein comprises an amino acid sequence at least about 60%, 65%, 70%, 75%, 80%, 85%, 90%, 95%, 98% or more homologous to a marker protein disclosed.

An isolated nucleic acid molecule encoding a protein homologous to a marker protein disclosed can be created by introducing one or more nucleotide substitutions, additions or deletions into the nucleotide sequence of the gene encoding the marker protein, such that one or more amino acid substitutions, additions or deletions are introduced into the encoded protein. Mutations can be introduced into the genes (*e.g.*, a gene set forth in Tables 1 and 4) by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art These families include amino acids with basic side chains (*e.g*., lysine, arginine, histidine), acidic side chains (*e.g*., aspartic acid, glutamic acid), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine). Alternatively, mutations can be introduced randomly along all or part of a coding sequence of a gene, such as by saturation mutagenesis, and the resultant mutants can be screened for biological activity to identify mutants that retain activity. Following mutagenesis, the encoded protein can be expressed recombinantly and the activity of the protein can be determined.

In yet another embodiment, the nucleic acid molecules described can be modified at the base moiety, sugar moiety or phosphate backbone to improve, *e.g*., the stability, hybridization, or solubility of the molecule. For example, the deoxyribose phosphate backbone of the nucleic acid molecules can be modified to generate peptide nucleic acids (see Hyrup B. et al. (1996) Bioorganic & Medicinal Chemistry 4 (1): 5-23). As used herein, the terms "peptide nucleic acids" or "PNAs" refer to nucleic acid mimics, *e.g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup B. *et al.* (1996) *supra;* Perry-O'Keefe et al. Proc. Natl. Acad. Sci. 93: 14670-675.

PNAs can be used in therapeutic and diagnostic applications. For example, PNAs can be used as antisense or antigene agents for sequence-specific modulation of gene expression by, for example, inducing transcription or translation arrest or inhibiting replication. PNAs of the nucleic acid molecules disclosed (*e.g*., a gene set forth in Tables 1-13) can also be used in the analysis of single base pair mutations in a gene, (*e.g*., by PNA-directed PCR clamping); as 'artificial restriction enzymes' when used in combination with other enzymes, (*e.g.,* S1 nucleases (Hyrup B. (1996) *supra*)); or as probes or primers for DNA sequencing or hybridization (Hyrup B*. et al.* (1996) *supra;* Perry-O'Keefe *supra*).

In another embodiment, PNAs can be modified, (*e.g*., to enhance their stability or cellular uptake), by attaching lipophilic or other helper groups to PNA, by the formation of PNA-DNA chimeras, or by the use of liposomes or other techniques of drug delivery known in the art. For example, PNA-DNA chimeras of the nucleic acid molecules disclosed can be generated which may combine the advantageous properties of PNA and DNA. Such chimeras allow DNA recognition enzymes, (*e.g*., RNAse H and DNA polymerases), to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup B. (1996) *supra*). The synthesis of PNA-DNA chimeras can be performed as described in Hyrup B. (1996) *supra* and Finn P.J. et al. (1996) Nucleic Acids Res. 24 (17): 3357-63. For example, a DNA chain can be synthesized on a solid support using standard phosphoramidite coupling chemistry and modified nucleoside analogs, *e.g*., 5'-(4-methoxytrityl)amino-5'-deoxythymidine phosphoramidite, can be used as a between the PNA and the 5' end of DNA (Mag, M. et al. (1989) Nucleic Acid Res. 17: 5973-88). PNA monomers are then coupled in a stepwise manner to produce a chimeric molecule with a 5' PNA segment and a 3' DNA segment (Finn P.J. *et al.* (1996) *supra*). Alternatively, chimeric molecules can be synthesized with a 5' DNA segment and a 3' PNA segment (Peterser, K.H. et al. (1975) Bioorganic Med. Chem. Lett. 5: 1119-11124).

In other embodiments, the oligonucleotide may include other appended groups such as peptides (e.g., for targeting host cell receptors *in vivo),* or agents facilitating transport across the cell membrane (see, *e.g*., Letsinger et al. (1989) Proc. Natl. Acad. Sci. USA 86:6553-6556; Lemaitre et al. (1987) Proc. Natl. Acas Sci. USA 84:648-652; PCT Publication No. W088/09810) or the blood-brain barrier (see, *e.g*., PCT Publication No. W089/10134). In addition, oligonucleotides can be modified with hybridization-triggered cleavage agents (See, *e.g.,* Krol et al. (1988) Bio-Techniques 6:958-976) or intercalating agents. (See, *e.g*., Zon (1988) Pharm. Res. 5:539-549). To this end, the oligonucleotide may be conjugated to another molecule, (*e.g*., a peptide, hybridization triggered cross-linking agent, transport agent, or hybridization-triggered cleavage agent). Finally, the oligonucleotide may be detectably labeled, either such that the label is detected by the addition of another reagent (*e.g*., a substrate for an enzymatic label), or is detectable immediately upon hybridization of the nucleotide (*e.g*., a radioactive label or a fluorescent label (*e.g*., a molecular beacon, as described in U.S. Patent 5,876,930.

### II. Isolated Proteins and Antibodies

One aspect pertains to isolated marker proteins, and biologically active portions thereof, as well as polypeptide fragments suitable for use as immunogens to raise anti-marker protein antibodies. In one embodiment, native marker proteins can be isolated from cells or tissue sources by an appropriate purification scheme using standard protein purification techniques. In another embodiment, marker proteins are produced by recombinant DNA techniques. Alternative to recombinant expression, a marker protein or polypeptide can be synthesized chemically using standard peptide synthesis techniques.

An "isolated" or "purified" protein or biologically active portion thereof is substantially free of cellular material or other contaminating proteins from the cell or tissue source from which the marker protein is derived, or substantially free from chemical precursors or other chemicals when chemically synthesized. The language "substantially free of cellular material" includes preparations of marker protein in which the protein is separated from cellular components of the cells from which it is isolated or recombinantly produced. In one embodiment, the language "substantially free of cellular material" includes preparations of marker protein having less than about 30% (by dry weight) of non-marker protein (also referred to herein as a "contaminating protein"), more preferably less than about 20% of non-marker protein, still more preferably less than about 10% of non-marker protein, and most preferably less than about 5% non-marker protein. When the marker protein or biologically active portion thereof is recombinantly produced, it is also preferably substantially free of culture medium, *i.e*., culture medium represents less than about 20%, more preferably less than about 10%, and most preferably less than about 5% of the volume of the protein preparation.

The language "substantially free of chemical precursors or other chemicals" includes preparations of marker protein in which the protein is separated from chemical precursors or other chemicals which are involved in the synthesis of the protein. In one embodiment, the language "substantially free of chemical precursors or other chemicals" includes preparations of protein having less than about 30% (by dry weight) of chemical precursors or non-protein chemicals, more preferably less than about 20% chemical precursors or non-protein chemicals, still more preferably less than about 10% chemical precursors or non-protein chemicals, and most preferably less than about 5% chemical precursors or non-protein chemicals.

As used herein, a "biologically active portion" of a marker protein includes a fragment of a marker protein comprising amino acid sequences sufficiently homologous to or derived from the amino acid sequence of the marker protein, which include fewer amino acids than the full length marker proteins, and exhibit at least one activity of a marker protein. Typically, biologically active portions comprise a domain or motif with at least one activity of the marker protein. A biologically active portion of a marker protein can be a polypeptide which is, for example, 10, 25, 50, 100, 200 or more amino acids in length. Biologically active portions of a marker protein can be used as targets for developing agents which modulate a marker protein-mediated activity.

In a preferred embodiment, marker protein is encoded by a gene set forth in Tables 1 and 4. In other embodiments, the marker protein is substantially homologous to a marker protein encoded by a gene set forth in Tables 1-13, and retains the functional activity of the marker protein, yet differs in amino acid sequence due to natural allelic variation or mutagenesis, as described in detail in subsection I above. Accordingly, in another embodiment, the marker protein is a protein which comprises an amino acid sequence at least about 60%, 65%, 70%, 75%, 80%, 85%, 90%, 95%, 98% or more homologous to the amino acid sequence encoded by a gene set forth in Tables 1 and 4.

To determine the percent identity of two amino acid sequences or of two nucleic acid sequences, the sequences are aligned for optimal comparison purposes (*e.g*., gaps can be introduced in one or both of a first and a second amino acid or nucleic acid ' sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%; 80%, or 90% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman and Wunsch (J. Mol: Biol. (48):444-453 (1970)) algorithm which has been incorporated into the GAP program in the GCG software package (available at http://www.gcg.com), using either a Blossom 62 matrix or a PAM250 matrix, and a gap weight of 16,14, 12,10, 8, 6, or 4 and a length weight of 1, 2, 3, 4, 5, or 6. In yet another preferred embodiment, the percent identity between two nucleotide sequences is determined using the GAP program in the GCG software package (available at http:/Avww.gcg.com), using a NWSgapdna.CMP matrix and a gap weight of 40,50,60, 70, or 80 and a length weight of 1, 2, 3, 4, 5, or 6. In another embodiment, the percent identity between two amino acid or nucleotide sequences is determined using the algorithm of E. Meyers and W. Miller (CABIOS, 4:11-17 (1989)) which has been incorporated into the ALIGN program (version 2.0), using a PAM120 weight residue table, a gap length penalty of 12 and a gap penalty of 4.

The nucleic acid and protein sequences disclosed can further be used as a "query sequence" to perform a search against public databases to, for example, identify other family members or related sequences. Such searches can be performed using the NBLAST and XBLAST programs (version 2.0) *of* Altschul, et al. (1990) J. Mol. Biol. 215:403-10*.* BLAST nucleotide searches can be performed with the NBLAST program, score =100, wordlength =12 to obtain nucleotide sequences homologous to nucleic acid molecules of the invention. BLAST protein searches can be performed with the XBLAST program, score = 50, wordlength = 3 to obtain amino acid sequences homologous to marker protein molecules of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (*e.g*., XBLAST and NBLAST) can be used. See http://www.ncbi.nlm.nih.gov.

Chimeric or fusion marker proteins are also provided. As used herein, a marker "chimeric protein" or "fusion protein" comprises a marker polypeptide operatively linked to a non-marker polypeptide. An "marker polypeptide" includes a polypeptide having an amino acid sequence encoded by a gene set forth in Tables 1 and 4, whereas a "non-marker polypeptide" includes a polypeptide having an amino acid sequence corresponding to a protein which is not substantially homologous to the marker protein, *e.g*., a protein which is different from marker protein and which is derived from the same or a different organism. Within a marker fusion protein the polypeptide can correspond to all or a portion of a marker protein. In a preferred embodiment, a marker fusion protein comprises at least one biologically active portion of a marker protein. Within the fusion protein, the term "operatively linked" is intended to indicate that the marker polypeptide and the non-marker polypeptide are fused in-frame to each other. The non-marker polypeptide can be fused to the N-terminus or C-terminus of the marker polypeptide.

For example, in one embodiment, the fusion protein is a GST-marker fusion protein in which the marker sequences are fused to the C-terminus of the GST sequences. Such fusion proteins can facilitate the purification of recombinant marker proteins.

In another embodiment, the fusion protein is a marker protein containing a heterologous signal sequence at its N-terminus. In certain host cells (*e.g.*, mammalian host cells), expression and/or secretion of marker proteins can be increased through use of a heterologous signal sequence. Such signal sequences are well known in the art.

The marker fusion proteins disclosed can be incorporated into pharmaceutical compositions and administered to a subject *in vivo,* as described herein. The marker fusion proteins can be used to affect the bioavailability of a marker protein substrate. Use of marker fusion proteins may be useful therapeutically for the treatment of disorders (*e.g.*, type I diabetes or an NKT-associated condition) caused by, for example, (i) aberrant modification or mutation of a gene encoding a marker protein; (ii) mis-regulation of the marker protein-encoding gene; and (iii) aberrant post-translational modification of a marker protein.

Moreover, the marker-fusion proteins disclosed can be used as immunogens to produce anti-marker protein antibodies in a subject, to purify marker protein ligands and in screening assays to identify molecules which inhibit the interaction of a marker protein with a marker protein substrate.

Preferably, a marker chimeric or fusion protein disclosed is produced by standard recombinant DNA techniques. For example, DNA fragments coding for the different polypeptide sequences are ligated together in-frame in accordance with conventional techniques, for example by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, for example, Current Protocols in Molecular Biology, eds. Ausubel et al. John Wiley & Sons: 1992). Moreover, many expression vectors are commercially available that already encode a fusion moiety (e.g., a GST polypeptide). A marker protein-encoding nucleic acid can be cloned into such an expression vector such that the fusion moiety is linked in-frame to the marker protein.

A signal sequence can be used to facilitate secretion and isolation of the secreted protein or other proteins of interest. Signal sequences are typically characterized by a core of hydrophobic amino acids which are generally cleaved from the mature protein during secretion in one or more cleavage events. Such signal peptides contain processing sites that allow cleavage of the signal sequence from the mature proteins as they pass through the secretory pathway. Thus, polypeptides having a signal sequence, as well as polypeptides from which the signal sequence has been proteolytically cleaved (*i.e.*, the cleavage products) are described. In one embodiment, a nucleic acid sequence encoding a signal sequence can be operably linked in an expression vector to a protein of interest, such as a protein which is ordinarily not secreted or is otherwise difficult to isolate. The signal sequence directs secretion of the protein, such as from a eukaryotic host into which the expression vector is transformed, and the signal sequence is subsequently or concurrently cleaved. The protein can then be readily purified from the extracellular medium by art recognized methods. Alternatively, the signal sequence can be linked to the protein of interest using a sequence which facilitates purification, such as with a GST domain.

Variants of the marker proteins disclosed which function as either agonists (mimetics) or as antagonists to the marker proteins are described. Variants of the marker proteins can be generated by mutagenesis, *e.g.*, discrete point mutation or truncation of a marker protein. An agonist of the marker proteins can retain substantially the same, or a subset, of the biological activities of the naturally occurring form of a marker protein. An antagonist of a marker protein can inhibit one or more of the activities of the naturally occurring form of the marker protein by, for example, competitively modulating an activity of a marker protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the marker protein.

Variants of a marker protein which function as either marker protein agonists (mimetics) or as marker protein antagonists can be identified by screening combinatorial libraries of mutants, *e.g.*, truncation mutants, of a marker protein for marker protein agonist or antagonist activity. In one embodiment, a variegated library of marker protein variants is generated by combinatorial mutagenesis at the nucleic acid level and is encoded by a variegated gene library. A variegated library of marker protein variants can be produced by, for example, enzymatically ligating a mixture of synthetic oligonucleotides into gene sequences such that a degenerate set of potential marker protein sequences is expressible as individual polypeptides, or alternatively, as a set of larger fusion proteins (*e.g.*, for phage display) containing the set of marker protein sequences therein. There are a variety of methods which can be used to produce libraries of potential marker protein variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential marker protein sequences. Methods for synthesizing degenerate oligonucleotides are known in the art (see, *e.g.,* Narang, S.A. (1983) Tetrahedron 39:3; Itakura et al. (1984) Annu. Rev. Biochem. 53:323; Itakura et al. (1984) Science 198:1056; Ike et al. (1983) Nucleic Acid Res. 11:477).

In addition, libraries of fragments of a protein coding sequence corresponding to a marker protein disclosed can be used to generate a variegated population of marker protein fragments for screening and subsequent selection of variants of a marker protein. In one embodiment, a library of coding sequence fragments can be generated by treating a double stranded PCR fragment of a marker protein coding sequence with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with S1 nuclease, and ligating the resulting fragment library into an expression vector. By this method, an expression library can be derived which encodes N-terminal, C-terminal and internal fragments of various sizes of the marker protein.

Several techniques are known in the art for screening gene products of combinatorial libraries made by point mutations or truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique which enhances the frequency of functional mutants in the libraries, can be used in combination with the screening assays to identify marker variants (Arkin and Yourvan (1992) Proc. Natl. Acad. Sci. USA 89:7811-7815; Delgrave et al. (1993) Protein Engineering 6(3):327-331).

An isolated marker protein, or a portion or fragment thereof, can be used as an immunogen to generate antibodies that bind marker proteins using standard techniques for polyclonal and monoclonal antibody preparation. A full-length marker protein can be used or, alternatively, antigenic peptide fragments of these proteins are provided for use as immunogens. The antigenic peptide of a marker protein comprises at least 8 amino acid residues of an amino acid sequence encoded by a gene set forth in Tables 1-13, and encompasses an epitope of a marker protein such that an antibody raised against the peptide forms a specific immune complex with the marker protein. Preferably, the antigenic peptide comprises at least 10 amino acid residues, more preferably at least 15 amino acid residues, even more preferably at least 20 amino acid residues, and most preferably at least 30 amino acid residues.

Preferred epitopes encompassed by the antigenic peptide are regions of the marker protein that are located on the surface of the protein, *e.g.*, hydrophilic regions, as well as regions with high antigenicity.

A marker protein immunogen typically is used to prepare antibodies by immunizing a suitable subject, (*e.g.*, rabbit, goat, mouse or other mammal) with the immunogen. An appropriate immunogenic preparation can contain, for example, recombinantly expressed marker protein or a chemically synthesized marker polypeptide. The preparation can further include an adjuvant, such as Freund's complete or incomplete adjuvant, or similar immunostimulatory agent. Immunization of a suitable subject with an immunogenic marker protein preparation induces a polyclonal anti-marker protein antibody response.

Accordingly, another aspect pertains to anti-marker protein antibodies. The term "antibody" as used herein includes immunoglobulin molecules and immunologically active portions of immunoglobulin molecules, *i.e.*, molecules that contain an antigen binding site which specifically binds (immunoreacts with) an antigen, such as a marker protein. Examples of immunologically active portions of immunoglobulin molecules include F(ab) and F(ab')₂ fragments which can be generated by treating the antibody with an enzyme such as pepsin. Polyclonal and monoclonal antibodies that bind to marker proteins are provided. The term "monoclonal antibody" or "monoclonal antibody composition", as used herein, includes a population of antibody molecules that contain only one species of an antigen binding site capable of immunoreacting with a particular epitope. A monoclonal antibody composition thus typically displays a single binding affinity for a particular marker protein with which it immunoreacts.

Polyclonal anti-marker protein antibodies can be prepared as described above by immunizing a suitable subject with a marker protein disclosed. The anti-marker protein antibody titer in the immunized subject can be monitored over time by standard techniques, such as with an enzyme linked immunosorbent assay (ELISA) using immobilized marker protein. If desired, the antibody molecules directed against marker proteins can be isolated from the mammal (*e.g.*, from the blood) and further purified by well known techniques, such as protein A chromatography, to obtain the IgG fraction. At an appropriate time after immunization, *e.g.*, when the anti-marker protein antibody titers are highest, antibody-producing cells can be obtained from the subject and used to prepare monoclonal antibodies by standard techniques, such as the hybridoma technique originally described by Kohler and Milstein (1975) Nature 256:495-497) (see also, Brown et al. (1981) J. Immunol. 127:539-46; Brown et al. (1980) J. Biol. Chem .255:4980-83; Yeh et al. (1976) Proc. Natl. Acad. Sci. USA 76:2927-31; and Yeh et al. (1982) Int. J. Cancer 29:269-75), the more recent human B cell hybridoma technique (Kozbor et al. (1983) Immunol Today 4:72), the EBV-hybridoma technique (Cole et al. (1985), Monoclonal Antibodies and Cancer Therapy, Alan R. Liss, Inc., pp. 77-96) or trioma techniques. The technology for producing monoclonal antibody hybridomas is well known (see generally R. H. Kenneth, in Monoclonal Antibodies: A New Dimension In Biological Analyses, Plenum Publishing Corp., New York, New York (1980); E. A. Lerner (1981) Yale J. Biol. Med., 54:387-402; M. L. Gefter et al. (1977) Somatic Cell Genet. 3:231-36). Briefly, an immortal cell line (typically a myeloma) is fused to lymphocytes (typically splenocytes) from a mammal immunized with a marker protein immunogen as described above, and the culture supernatants of the resulting hybridoma cells are screened to identify a hybridoma producing a monoclonal antibody that binds to a marker protein of the invention.

Any of the many well known protocols used for fusing lymphocytes and immortalized cell lines can be applied for the purpose of generating an anti-marker protein monoclonal antibody (see, *e.g.,* G. Galfre et al. (1977) Nature 266:55052; Gefter *et al. Somatic Cell Genet.,* cited *supra;* Lerner, *Yale J. Biol. Med.,* cited *supra;* Kenneth, *Monoclonal Antibodies,* cited *supra).* Moreover, the ordinarily skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g.*, the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind to a marker protein, *e.g.*, using a standard ELISA assay.

Alternative to preparing monoclonal antibody-secreting hybridomas, a monoclonal anti-marker protein antibody can be identified and isolated by screening a recombinant combinatorial immunoglobulin library (*e.g.*, an antibody phage display library) with marker protein to thereby isolate immunoglobulin library members that bind to a marker protein. Kits for generating and screening phage display libraries are commercially available (*e.g.*, the Pharmacia *Recombinant Phage Antibody System,* Catalog No. 27-9400-01; and the Stratagene *SurfZAP*™ *Phage Display Kit,* Catalog No. 240612). Additionally, examples of methods and reagents particularly amenable for use in generating and screening antibody display library can be found in, for example, Ladner et al. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) Bio/Technology 9:1370-1372; Hay et al. (1992) Hum. Antibod. Hybridomas 3:81-85; Huse et al. (1989) Science 246:1275-1281; Griffiths et al. (1993) EMBO J 12:725-734; Hawkins et al. (1992) J.. Mol. Biol. 226:889-896; Clarkson et al. (1991) Nature 352:624-628; Gram et al. (1992) Proc. Natl. Acad. Sci. USA 89:3576-3580; Garrad et al. (1991) Bio/Technology 9:1373-1377; Hoogenboom et al. (1991) Nuc. Acid Res. 19:4133-4137; Barbas et al. (1991) Proc. Natl. Acad. Sci. USA 88:7978-7982; and McCafferty et al. Nature (1990) 348:552-554.

Additionally, recombinant anti-marker protein antibodies, such as chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson *et al.* International Application No. PCT/US86/02269; Akira, *et al.* European Patent Application 184,187; Taniguchi, M., European Patent Application 171,496; Morrison *et al.* European Patent Application 173,494; Neuberger et al. PCT International Publication No. WO 86/01533; Cabilly et al. U.S. Patent No. 4,816,567; Cabilly *et al.* European Patent Application 125,023; Better et al. (1988) Science 240:1041-1043; Liu et al. (1987) Proc. Natl. Acad. Sci. USA 84:3439-3443; Liu et al. (1987) J. Immunol. 139:3521-3526; Sun et al. (1987) Proc. Natl. Acad. Sci. USA 84:214-218; Nishimura et al. (1987) Canc. Res. 47:999-1005; Wood et al. (1985) Nature 314:446-449; and Shaw et al. (1988) J. Natl. Cancer Inst. 80:1553-1559); Morrison, S. L. (1985) Science 229:1202-1207; Oi et al. (1986) BioTechniques 4:214; Winter U.S. Patent 5,225,539; Jones et al. (1986) Nature 321:552-525; Verhoeyan et al. (1988) Science 239:1534; and Beidler et al. (1988) J. Immunol. 141:4053-4060.

Completely human antibodies are particularly desirable for therapeutic treatment of human subjects. Such antibodies can be produced using transgenic mice which are incapable of expressing endogenous immunoglobulin heavy and light chains genes, but which can express human heavy and light chain genes. The transgenic mice are immunized in the normal fashion with a selected antigen, *e.g.*, all or a portion of a polypeptide corresponding to a marker of the invention. Monoclonal antibodies directed against the antigen can be obtained using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA and IgE antibodies. For an overview of this technology for producing human antibodies, see Lonberg and Huszar (1995) Int. Rev. Immunol. 13:65-93). For a detailed discussion of this technology for producing human antibodies and human monoclonal antibodies and protocols for producing such antibodies, see, *e.g.*, U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA), can be engaged to provide human antibodies directed against a selected antigen using technology similar to that described above.

Completely human antibodies which recognize a selected epitope can be generated using a technique referred to as "guided selection." In this approach a selected non-human monoclonal antibody, *e.g.*, a murine antibody, is used to guide the selection of a completely human antibody recognizing the same epitope (Jespers et al., 1994, Bio/technology 12:899-903).

An anti-marker protein antibody (*e.g.*, monoclonal antibody) can be used to isolate a marker protein disclosed by standard techniques, such as affinity chromatography or immunoprecipitation. An anti-marker protein antibody can facilitate the purification of natural marker proteins from cells and of recombinantly produced marker proteins expressed in host cells. Moreover, an anti-marker protein antibody can be used to detect marker protein (*e.g.*, in a cellular lysate or cell supernatant) in order to evaluate the abundance and pattern of expression of the marker protein. Anti-marker protein antibodies can be used diagnostically to monitor protein levels in tissue as part of a clinical testing procedure, *e.g.*, to, for example, determine the efficacy of a given treatment regimen. Detection can be facilitated by coupling *(i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, -galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### III. Recombinant Expression Vectors and Host Cells

Another aspect pertains to vectors, preferably expression vectors, containing a nucleic acid encoding a marker protein of the invention (or a portion thereof). As used herein, the term "vector" includes a nucleic acid molecule capable of transporting another nucleic acid to which it has been linked. One type of vector is a "plasmid", which includes a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, other forms of expression vectors, such as viral vectors (*e.g.*, replication defective retroviruses, adenoviruses and adeno-associated viruses), which serve equivalent functions, are also described.

The recombinant expression vectors described comprise a nucleic acid disclosed in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (*e.g.*, in an *in vitro* transcription/translation system or in a host cell when the vector is introduced into the host cell). The term "regulatory sequence" is intended to include promoters, enhancers and other expression control elements (*e.g.*, polyadenylation signals). Such regulatory sequences are described, for example, in Goeddel; Gene Expression Technology: Methods in Enzymology 185, Academic Press, San Diego, CA (1990). Regulatory sequences include those which direct constitutive expression of a nucleotide sequence in many types of host cells and those which direct expression of the nucleotide sequence only in certain host cells (*e.g.*, tissue-specific regulatory sequences). It will be appreciated by those skilled in the art that the design of the expression vector can depend on such factors as the choice of the host cell to be transformed, the level of expression of protein desired, and the like. The expression vectors disclosed can be introduced into host cells to thereby produce proteins or peptides, including fusion proteins or peptides, encoded by nucleic acids as described herein (*e.g.*, marker proteins, mutant forms of marker proteins, fusion proteins, and the like).

The recombinant expression vectors disclosed can be designed for expression of marker proteins in prokaryotic or eukaryotic cells. For example, marker proteins can be expressed in bacterial cells such as *E. coli,* insect cells (using baculovirus expression vectors) yeast cells or mammalian cells. Suitable host cells are discussed further in Goeddel, Gene Expression Technology: Methods in Enzymology 185, Academic Press, San Diego, CA (1990). Alternatively, the recombinant expression vector can be transcribed and translated *in vitro,* for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith, D.B. and Johnson, K.S. (1988) Gene 67:31-40), pMAL (New England Biolabs, Beverly, MA) and pRIT5 (Pharmacia, Piscataway, NJ) which fuse glutathione S-transferase (GST), maltose E binding protein, or protein A, respectively, to the target recombinant protein.

Purified fusion proteins can be utilized in marker activity assays, (*e.g.*, direct assays or competitive assays described in detail below), or to generate antibodies specific for marker proteins, for example.

Examples of suitable inducible non-fusion *E. coli* expression vectors include pTrc (Amann et al., (1988) Gene 69:301-315) and pET 11d (Studier et αl., Gene Expression Technology: Methods in Enzymology 185, Academic Press, San Diego, California (1990) 60-89). Target gene expression from the pTrc vector relies on host RNA polymerase transcription from a hybrid trp-lac fusion promoter. Target gene expression from the pET 11d vector relies on transcription from a T7 gn10-lac fusion promoter mediated by a coexpressed viral RNA polymerase (T7 gnl). This viral polymerase is supplied by host strains BL21(DE3) or HMS174(DE3) from a resident prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, S., Gene Expression Technology: Methods in Enzymology 185, Academic Press, San Diego, California (1990) 119-128). Another strategy is to alter the nucleic acid sequence of the nucleic acid to be inserted into an expression vector so that the individual codons for each amino acid are those preferentially utilized in *E. coli (*Wada et αl., (1992) Nucleic Acids Res. 20:2111-2118). Such alteration of nucleic acid sequences disclosed can be carried out by standard DNA synthesis techniques.

In another embodiment, the marker protein expression vector is a yeast expression vector. Examples of vectors for expression in yeast S. *cerevisiae* include pYepSec1 *(*Baldari, et al., (1987) Embo J. 6:229-234), pMFa (Kurjan and Herskowitz, (1982) Cell 30:933-943), pJRY88 *(*Schultz et αl., (1987) Gene 54:113-123), pYES2 (Invitrogen Corporation, San Diego, CA), and picZ (InVitrogen Corp, San Diego, CA).

Alternatively, marker proteins disclosed can be expressed in insect cells using baculovirus expression vectors. Baculovirus vectors available for expression of proteins in cultured insect cells *(e.g.,* Sf 9 cells) include the pAc series (Smith et al. (1983) Mol. Cell Biol. 3:2156-2165) and the pVL series (Lucklow and Summers (1989) Virology 170:31-39).

In yet another embodiment, a nucleic acid disclosed is expressed in mammalian cells using a mammalian expression vector. Examples of mammalian expression vectors include pCDM8 (Seed, B. (1987) Nature 329:840) and pMT2PC (Kaufman et al. (1987) EMBO J. 6:187-195). When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989.

In another embodiment, the recombinant mammalian expression vector is capable of directing expression of the nucleic acid preferentially in a particular cell type (*e.g.*, tissue-specific regulatory elements are used to express the nucleic acid). Tissue-specific regulatory elements are known in the art. Non-limiting examples of suitable tissue-specific promoters include the albumin promoter (liver-specific; Pinkert et αl. (1987) Genes Dev. 1:268-277), lymphoid-specific promoters (Calame and Eaton (1988) Adv. Immunol. 43:235-275), in particular promoters of T cell receptors (Winoto and Baltimore (1989) EMBO J. 8:729-733) and immunoglobulins (Banerji et αl. (1983) Cell 33:729-740; Queen and Baltimore (1983) Cell 33:741-748), neuron-specific promoters (*e.g.*, the neurofilament promoter; Byrne and Ruddle (1989) Proc. Natl. Acad. Sci. USA 86:5473-5477), pancreas-specific promoters (Edlund et αl. (1985) Science 230:912-916), and mammary gland-specific promoters (*e.g.*, milk whey promoter; U.S. Patent No. 4,873,316 and European Application Publication No. 264,166). Developmentally-regulated promoters are also encompassed, for example the murine hox promoters (Kessel and Gruss (1990) Science 249:374-379) and the α-fetoprotein promoter (Campes and Tilghman (1989) Genes Dev. 3:537-546).

Further, a recombinant expression vector comprising a DNA molecule disclosed cloned into the expression vector in an antisense orientation is provided. That is, the DNA molecule is operatively linked to a regulatory sequence in a manner which allows for expression (by transcription of the DNA molecule) of an RNA molecule which is antisense to mRNA corresponding to a gene described (*e.g.*, a gene set forth in Tables 1-13). Regulatory sequences operatively linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes see Weintraub, H. et al., Antisense RNA as a molecular tool for genetic analysis, Reviews - Trends in Genetics, Vol. 1(1) 1986.

Another aspect pertains to host cells into which a nucleic acid molecule disclosed is introduced, *e.g.*, a gene set forth in Tables 1-13 within a recombinant expression vector or a nucleic acid molecule disclosed containing sequences which allow it to homologously recombine into a specific site of the host cell's genome. The terms "host cell" and "recombinant host cell" are used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, a marker protein disclosed can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e.g.*, DNA) into a host cell, including calcium phosphate or calcium chloride co-precipitation, DEAE-dextran-mediated transfection, lipofection, or electroporation. Suitable methods for transforming or transfecting host cells can be found in Sambrook, et αl. (Molecular Cloning: A Laboratory Manual. 2nd, ed., Cold Spring Harbor Laboratory, Cold Spring Harbor Laboratory Press, Cold Spring Harbor, NY, 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g.*, resistance to antibiotics) is generally introduced into the host cells along with the gene of interest. Preferred selectable markers include those which confer resistance to drugs, such as G418, hygromycin and methotrexate. Nucleic acid encoding a selectable marker can be introduced into a host cell on the same vector as that encoding a marker protein or can be introduced on a separate vector. Cells stably transfected with the introduced nucleic acid can be identified by drug selection (*e.g.*, cells that have incorporated the selectable marker gene will survive, while the other cells die).

A host cell disclosed, such as a prokaryotic or eukaryotic host cell in culture, can be used to produce *(i.e.,* express) a marker protein. Accordingly, methods for producing a marker protein using the host cells disclosed is provided. In one embodiment, the method comprises culturing the host cell (into which a recombinant expression vector encoding a marker protein has been introduced) in a suitable medium such that a marker protein is produced. In another embodiment, the method further comprises isolating a marker protein from the medium or the host cell.

The host cells can also be used to produce non-human transgenic animals. For example, in one embodiment, a host cell is a fertilized oocyte or an embryonic stem cell into which marker-protein-coding sequences have been introduced. Such host cells can then be used to create non-human transgenic animals in which exogenous sequences encoding a marker protein disclosed have been introduced into their genome or homologous recombinant animals in which endogenous sequences encoding the marker proteins disclosed have been altered. Such animals are useful for studying the function and/or activity of a marker protein and for identifying and/or evaluating modulators of marker protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA which is integrated into the genome of a cell from which a transgenic animal develops and which remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous gene (*e.g.*, a gene set forth in Tables 1-13) has been altered by homologous recombination between the endogenous gene and an exogenous DNA molecule introduced into a cell of the animal, *e.g.*, an embryonic cell of the animal, prior to development of the animal.

A transgenic animal can be created by introducing a marker-encoding nucleic acid into the male pronuclei of a fertilized oocyte, *e.g.*, by microinjection, retroviral infection, and allowing the oocyte to develop in a pseudopregnant female foster animal. Intronic sequences and polyadenylation signals can also be included in the transgene to increase the efficiency of expression of the transgene. A tissue-specific regulatory sequence(s) can be operably linked to a transgene to direct expression of a marker protein to particular cells. Methods for generating transgenic animals via embryo manipulation and microinjection, particularly animals such as mice, have become conventional in the art and are described, for example, in U.S. Patent Nos. 4,736,866 and 4,870,009, both by Leder et αl*.,* U.S. Patent No. 4,873,191 by Wagner et αl. and in Hogan, B., Manipulating the Mouse Embryo, (Cold Spring Harbor Laboratory Press, Cold Spring Harbor, N.Y., 1986). Similar methods are used for production of other transgenic animals. A transgenic founder animal can be identified based upon the presence of a transgene disclosed in its genome and/or expression of mRNA corresponding to a gene of the invention in tissues or cells of the animals. A transgenic founder animal can then be used to breed additional animals carrying the transgene. Moreover, transgenic animals carrying a transgene encoding a marker protein can further be bred to other transgenic animals carrying other transgenes.

To create a homologous recombinant animal, a vector is prepared which contains at least a portion of a gene disclosed into which a deletion, addition or substitution has been introduced to thereby alter, *e.g.*, functionally disrupt, the gene. The gene can be a human gene, but more preferably, is a non-human homologue of a human gene disclosed (*e.g.*, a gene set forth in Tables 1-13). For example, a mouse gene can be used to construct a homologous recombination nucleic acid molecule, *e.g.*, a vector, suitable for altering an endogenous gene in the mouse genome. In a preferred embodiment, the homologous recombination nucleic acid molecule is designed such that, upon homologous recombination, the endogenous gene is functionally disrupted *(i.e.,* no longer encodes a functional protein; also referred to as a "knock out" vector). Alternatively, the homologous recombination nucleic acid molecule can be designed such that, upon homologous recombination, the endogenous gene is mutated or otherwise altered but still encodes functional protein (*e.g.*, the upstream regulatory region can be altered to thereby alter the expression of the endogenous marker protein). In the homologous recombination nucleic acid molecule, the altered portion of the gene is flanked at its 5' and 3' ends by additional nucleic acid sequence of the gene to allow for homologous recombination to occur between the exogenous gene carried by the homologous recombination nucleic acid molecule and an endogenous gene in a cell, *e.g.*, an embryonic stem cell. The additional flanking nucleic acid sequence is of sufficient length for successful homologous recombination with the endogenous gene. Typically, several kilobases of flanking DNA (both at the 5' and 3' ends) are included in the homologous recombination nucleic acid molecule (see, *e.g.*, Thomas, K.R. and Capecchi, M. R. (1987) Cell 51:503 for a description of homologous recombination vectors). The homologous recombination nucleic acid molecule is introduced into a cell, *e.g.,* an embryonic stem cell line (*e.g.,* by electroporation) and cells in which the introduced gene has homologously recombined with the endogenous gene are selected (see *e.g.,* Li, E. et al. (1992) Cell 69:915). The selected cells can then injected into a blastocyst of an animal (*e.g.*, a mouse) to form aggregation chimeras (see *e.g.*, Bradley, A. in Teratocarcinomas and Embryonic Stem Cells: A Practical Approach, E.J. Robertson, ed. (IRL, Oxford, 1987) pp. 113-152). A chimeric embryo can then be implanted into a suitable pseudopregnant female foster animal and the embryo brought to term. Progeny harboring the homologously recombined DNA in their germ cells can be used to breed animals in which all cells of the animal contain the homologously recombined DNA by germline transmission of the transgene. Methods for constructing homologous recombination nucleic acid molecules, *e.g.*, vectors, or homologous recombinant animals are described further in Bradley, A. (1991) Current Opinion in Biotechnology 2:823-829 and in PCT International Publication Nos.: WO 90/11354 by Le Mouellec et al.*;* WO 91/01140 by Smithies et al.*;* WO 92/0968 by Zijlstra et al*.;* and WO 93/04169 by Berns et al.

In another embodiment, transgenic non-human animals can be produced which contain selected systems which allow for regulated expression of the transgene. One example of such a system is the *cre*/*loxP* recombinase system of bacteriophage P1. For a description of the *cre*/*loxP* recombinase system, see, *e.g.*, Lakso et αl. (1992) Proc. Natl. Acad. Sci. USA 89:6232-6236. Another example of a recombinase system is the FLP recombinase system of *Saccharomyces cerevisiae* (O'Gorman et αl. (1991) Science 251:1351-1355. If a *cre*/*loxP* recombinase system is used to regulate expression of the transgene, animals containing transgenes encoding both the *Cre* recombinase and a selected protein are required. Such animals can be provided through the construction of "double" transgenic animals, *e.g.*, by mating two transgenic animals, one containing a transgene encoding a selected protein and the other containing a transgene encoding a recombinase.

Clones of the non-human transgenic animals described herein can also be produced according to the methods described in Wilmut, I. et al. (1997) Nature 385:810-813 and PCT International Publication Nos. WO 97/07668 and WO 97/07669. In brief, a cell, *e.g.*, a somatic cell, from the transgenic animal can be isolated and induced to exit the growth cycle and enter Go phase. The quiescent cell can then be fused, *e.g.*, through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell, *e.g.*, the somatic cell, is isolated.

### V. Computer Readable Means and Arrays

Computer readable media comprising a marker(s) disclosed is also described. As used herein, "computer readable media" includes a medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. The skilled artisan will readily appreciate how any of the presently known computer readable mediums can be used to create a manufacture comprising computer readable medium having recorded thereon a marker disclosed.

As used herein, "recorded" includes a process for storing information on computer readable medium. Those skilled in the art can readily adopt any of the presently known methods for recording information on computer readable medium to generate manufactures comprising the markers disclosed.

A variety of data processor programs and formats can be used to store the marker information on computer readable medium. For example, the nucleic acid sequence corresponding to the markers can be represented in a word processing text file, formatted in commercially-available software such as WordPerfect and MicroSoft Word, or represented in the form of an ASCII file, stored in a database application, such as DB2, Sybase, Oracle, or the like. Any number of dataprocessor structuring formats (*e.g.*, text file or database) may be adapted in order to obtain computer readable medium having recorded thereon the markers disclosed.

By the markers disclosed in computer readable form, one can routinely access the marker sequence information for a variety of purposes. For example, one skilled in the art can use the nucleotide or amino acid sequences disclosed in computer readable form to compare a target sequence or target structural motif with the sequence information stored within the data storage means. Search means are used to identify fragments or regions of the sequences which match a particular target sequence or target motif.

An array comprising a marker(s) disclosed is also provided. The array can be used to assay expression of one or more genes in the array. In one embodiment, the array can be used to assay gene expression in a tissue to ascertain tissue specificity of genes in the array. In this manner, up to about 8600 genes can be simultaneously assayed for expression. This allows a profile to be developed showing a battery of genes specifically expressed in one or more tissues.

In addition to such qualitative determination, the disclosure the quantitation of gene expression. Thus, not only tissue specificity, but also the level of expression of a battery of genes in the tissue is ascertainable. Thus, genes can be grouped on the basis of their tissue expression *per se* and level of expression in that tissue. This is useful, for example, in ascertaining the relationship of gene expression between or among tissues. Thus, one tissue can be perturbed and the effect on gene expression in a second tissue can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell types, there is provided an assay to determine the molecular basis of the undesirable effect and thus provides the opportunity to co-administer a counteracting agent or otherwise treat the undesired effect. Similarly, even within a single cell type, undesirable biological effects can be determined at the molecular level Thus, the effects of an agent on expression of other than the target gene can be ascertained and counteracted.

In another embodiment, the array can be used to monitor the time course of expression of one or more genes in the array. This can occur in various biological contexts, as disclosed herein; for example development and differentiation, disease progression, *in vitro* processes, such a cellular transformation and senescence, autonomic neural and neurological processes, such as, for example, pain and appetite, and cognitive functions, such as learning or memory.

The array is also useful for ascertaining the effect of the expression of a gene on the expression of other genes in the same cell or in different cells. This provides, for example, for a selection of alternate molecular targets for therapeutic intervention if the ultimate or downstream target cannot be regulated.

The array is also useful for ascertaining differential expression patterns of one or more genes in normal and diseased cells. This provides a battery of genes that could serve as a molecular target for diagnosis or therapeutic intervention.

### VI. Predictive Medicine

The present disclosure pertains to the field of predictive medicine in which diagnostic assays, prognostic assays, pharmacogenetics and monitoring clinical trials are used for prognostic (predictive) purposes to thereby treat an individual prophylactically. Accordingly, one aspect relates to diagnostic assays for determining marker protein and/or nucleic acid expression as well as marker protein activity, in the context of a biological sample (*e.g.*, blood, serum, cells, tissue) to thereby determine whether an individual is afflicted with a disease or disorder, or is at risk of developing a disorder, associated with increased or decreased marker protein expression or activity. Prognostic (or predictive) assays for determining whether an individual is at risk of developing a disorder associated with marker protein, nucleic acid expression or activity are also provided. For example, the number of copies of a marker gene can be assayed in a biological sample. Such assays can be used for prognostic or predictive purposes to thereby phophylactically treat an individual prior to the onset of a disorder (*e.g.*, type I diabetes) characterized by or associated with marker protein, nucleic acid expression or activity.

Another aspect pertains to monitoring the influence of agents (*e.g.*, drugs, compounds) on the expression or activity of marker in clinical trials.

These and other agents are described in further detail in the following sections.

### 1. Diagnostic Assays

An exemplary method for detecting the presence or absence of marker protein or nucleic acid disclosed in a biological sample involves obtaining a biological sample from a test subject and contacting the biological sample with a compound or an agent capable of detecting the protein or nucleic acid (*e.g.*, mRNA, genomic DNA) that encodes the marker protein such that the presence of the marker protein or nucleic acid is detected in the biological sample. A preferred agent for detecting mRNA or genomic DNA corresponding to a marker gene or protein disclosed is a labeled nucleic acid probe capable of hybridizing to a mRNA or genomic DNA disclosed. Suitable probes for use in the diagnostic assays disclosed are described herein.

A preferred agent for detecting marker protein is an antibody capable of binding to marker protein, preferably an antibody with a detectable label. Antibodies can be polyclonal, or more preferably, monoclonal. An intact antibody, or a fragment thereof (*e.g.*, Fab or F(ab')₂) can be used. The term "labeled", with regard to the probe or antibody, is intended to encompass direct labeling of the probe or antibody by coupling *(i.e.,* physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. The term "biological sample" is intended to include tissues, cells and biological fluids isolated from a subject, as well as tissues, cells and fluids present within a subject. That is, the detection method disclosed can be used to detect marker mRNA, protein, or genomic DNA in a biological sample *in vitro* as well as *in vivo.* For example, *in vitro* techniques for detection of marker mRNA include Northern hybridizations and *in situ* hybridizations. *In vitro* techniques for detection of marker protein include enzyme linked immunosorbent assays (ELISAs), Western blots, immunoprecipitations and immunofluorescence. *In vitro* techniques for detection of marker genomic DNA include Southern hybridizations. Furthermore, *in vivo* techniques for detection of marker protein include introducing into a subject a labeled anti-marker antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a serum sample isolated by conventional means from a subject.

In another embodiment, the methods further involve obtaining a control biological sample (*e.g.*, nondiabetic tissue) from a control subject, contacting the control sample with a compound or agent capable of detecting marker protein, mRNA, or genomic DNA, such that the presence of marker protein, mRNA or genomic DNA is detected in the biological sample, and comparing the presence of marker protein, mRNA or genomic DNA in the control sample with the presence of marker protein, mRNA or genomic DNA in the test sample.

Kits for detecting the presence of marker in a biological sample are also provided. For example, the kit can comprise a labeled compound or agent capable of detecting marker protein or mRNA in a biological sample; means for determining the amount of marker in the sample; and means for comparing the amount of marker in the sample with a standard. The compound or agent can be packaged in a suitable container. The kit can further comprise instructions for using the kit to detect marker protein or nucleic acid.

### 2. Prognostic Assays

The diagnostic methods described herein can furthermore be utilized to identify subjects having or at risk of developing a disease or disorder associated with aberrant marker expression or activity. As used herein, the term "aberrant" includes a marker expression or activity which deviates from the wild type marker expression or activity. Aberrant expression or activity includes increased or decreased expression or activity, as well as expression or activity which does not follow the wild type developmental pattern of expression or the subcellular pattern of expression. For example, aberrant marker expression or activity is intended to include the cases in which a mutation in the marker gene causes the marker gene to be under-expressed or over-expressed and situations in which such mutations result in a non-functional marker protein or a protein which does not function in a wild-type fashion, *e.g.*, a protein which does not interact with a marker ligand or one which interacts with a non-marker protein ligand.

The assays described herein, such as the preceding diagnostic assays or the following assays, can be utilized to identify a subject having or at risk of developing a disorder associated with a misregulation in marker protein activity or nucleic acid expression, such as type I diabetes. Alternatively, the prognostic assays can be utilized to identify a subject having or at risk for developing a disorder associated with a misregulation in marker protein activity or nucleic acid expression, such as type I diabetes Thus, a method is provided for identifying a disease or disorder associated with aberrant marker expression or activity in which a test sample is obtained from a subject and marker protein or nucleic acid (*e.g.*, mRNA or genomic DNA) is detected, wherein the presence of marker protein or nucleic acid is diagnostic for a subject having or at risk of developing a disease or disorder associated with aberrant marker expression or activity. As used herein, a "test sample" includes a biological sample obtained from a subject of interest. For example, a test sample can be a biological fluid (*e.g.*, blood), cell samples, or tissue (*e.g.*, pancreatic tissue).

Furthermore, the prognostic assays described herein can be used to determine whether a subject can be administered an agent (*e.g.*, an agonist, antagonist, peptidomimetic, protein, peptide, nucleic acid, small molecule, or other drug candidate) to treat a disease or disorder associated with increased or decreased marker expression or activity. For example, such methods can be used to determine whether a subject can be effectively treated with an agent for a disorder such as type I diabetes. Thus, methods are provided for determining whether a subject can be effectively treated with an agent for a disorder associated with increased or decreased marker expression or activity in which a test sample is obtained and marker protein or nucleic acid expression or activity is detected (*e.g.,* wherein the abundance of marker protein or nucleic acid expression or activity is diagnostic for a subject that can be administered the agent to treat a disorder associated with increased or decreased marker expression or activity).

The methods disclosed can also be used to detect genetic alterations in a marker gene, thereby determining if a subject with the altered gene is at risk for a disorder characterized by misregulation in marker protein activity or nucleic acid expression, such as type I diabetes. In preferred embodiments, the methods include detecting, in a sample of cells from the subject, the presence or absence of a genetic alteration characterized by at least one of an alteration affecting the integrity of a gene encoding a marker-protein, or the mis-expression of the marker gene. For example, such genetic alterations can be detected by ascertaining the existence of at least one of 1) a deletion of one or more nucleotides from a marker gene; 2) an addition of one or more nucleotides to a marker gene; 3) a substitution of one or more nucleotides of a marker gene, 4) a chromosomal rearrangement of a marker gene; 5) an alteration in the level of a messenger RNA transcript of a marker gene, 6) aberrant modification of a marker gene, such as of the methylation pattern of the genomic DNA, 7) the presence of a non-wild type splicing pattern of a messenger RNA transcript of a marker gene, 8) a non-wild type level of a marker-protein, 9) allelic loss of a marker gene, and 10) inappropriate post-translational modification of a marker-protein. As described herein, there are a large number of assays known in the art which can be used for detecting alterations in a marker-gene. A preferred biological sample is a tissue (*e*.*g*., pancreatic tissue) or blood sample isolated by conventional means from a subject.

In certain embodiments, detection of the alteration involves the use of a probe/primer in a polymerase chain reaction (PCR) (see, e.g., U.S. Patent Nos. 4,683,195 and 4,683,202), such as anchor PCR or RACE PCR, or, alternatively, in a ligation chain reaction (LCR) (see, *e.g.,* Landegran et al. (1988) Science 241:1077-1080; and Nakazawa et al. (1994) Proc. Natl. Acad Sci. USA 91:360-364), the latter of which can be particularly useful for detecting point mutations in the marker-gene (see Abravaya et al. (1995) Nucleic Acids Res .23:675-682). This method can include the steps of collecting a sample of cells from a subject, isolating nucleic acid (*e.g*., genomic, mRNA or both) from the cells of the sample, contacting the nucleic acid sample with one or more primers which specifically hybridize to a marker gene under conditions such that hybridization and amplification of the marker-gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., (1990) Proc. Natl. Acad. Sci. USA 87:1874-1878), transcriptional amplification system (Kwoh, D.Y. et al., (1989) Proc. Natl. Acad. Sci. USA 86:1173-1177), Q-Beta Replicase (Lizardi, P.M. et al. (1988) Bio-Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a marker gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in a marker gene or a gene encoding a marker protein disclosed can be identified by hybridizing a sample and control nucleic acids, *e.g*., DNA or RNA, to high density arrays containing hundreds or thousands of oligonucleotides probes (Cronin, M.T. et al. (1996) Human Mutation 7: 244-255; Kozal, M.J. et al. (1996) Nature Medicine 2: 753-759). For example, genetic mutations in marker can be identified in two dimensional arrays containing light-generated DNA probes as described in Cronin, M.T. *et al. supra.* Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the marker gene and detect mutations by comparing the sequence of the sample marker with the corresponding wild-type (control) sequence. Examples of sequencing reactions include those based on techniques developed by Maxam and Gilbert ((1977) Proc. Natl. Acad. Sci. USA 74:560) or Sanger ((1977) Proc. Natl. Acad. Sci. USA 74:5463). It is also contemplated that any of a variety of automated sequencing procedures can be utilized when performing the diagnostic assays ((1995) Biotechniques 19:448), including sequencing by mass spectrometry (see, *e.g.,* PCT International Publication No. WO 94/16101; Cohen et al. (1996) Adv. Chromatogr. 36:127-162; and Griffin et al. (1993) Appl. Biochem. Biotechnol. 38:147-159).

Other methods for detecting mutations in the marker gene or gene encoding a marker protein disclosed include methods in which protection from cleavage agents is used to detect mismatched bases in RNA/RNA or RNA/DNA heteroduplexes (Myers et al. (1985) Science 230:1242). In general, the art technique of "mismatch cleavage" starts by providing heteroduplexes of formed by hybridizing (labeled) RNA or DNA containing the wild-type marker sequence with potentially mutant RNA or DNA obtained from a tissue sample. The double-stranded duplexes are treated with an agent which cleaves single-stranded regions of the duplex such as which will exist due to basepair mismatches between the control and sample strands. For instance, RNA/DNA duplexes can be treated with RNase and DNA/DNA hybrids treated with S 1 nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. See, for example, Cotton et al. (1988) Proc. Natl Acad Sci USA 85:4397; Saleeba et al. (1992) Methods Enzymol. 217:286-295. In a preferred embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in marker cDNAs obtained from samples of cells. For example, the mutY enzyme of *E. coli* cleaves A at G/A mismatches and the thymidine DNA glycosylase from HeLa cells cleaves T at G/T mismatches (Hsu et al. (1994) Carcinogenesis 15:1657-1662). According to an exemplary embodiment, a probe based on a marker sequence, *e*.*g*., a wild-type marker sequence, is hybridized to a cDNA or other DNA product from a test cell(s). The duplex is treated with a DNA mismatch repair enzyme, and the cleavage products, if any, can be detected from electrophoresis protocols or the like. See, for example, U.S. Patent No. 5,459,039.

In other embodiments, alterations in electrophoretic mobility will be used to identify mutations in marker genes or genes encoding a marker protein disclosed. For example, single strand conformation polymorphism (SSCP) may be used to detect differences in electrophoretic mobility between mutant and wild type nucleic acids *(*Orita et al. (1989) Proc Natl. Acad. Sci USA: 86:2766, see also Cotton (1993) Mutat. Res. 285:125-144; and Hayashi (1992) Genet. Anal. Tech. Appl. 9:73-79). Single-stranded DNA fragments of sample and control marker nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) Trends Genet 7:5).

In yet another embodiment the movement of mutant or wild-type fragments in polyacrylamide gels containing a gradient of denaturant is assayed using denaturing gradient gel electrophoresis (DGGE) (Myers et al. (1985) Nature 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) Biophys Chem 265:12753).

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions which permit hybridization only if a perfect match is found (Saiki et al. (1986) Nature 324:163); Saiki et al. (1989) Proc. Natl. Acad. Sci USA 86:6230). Such allele specific oligonucleotides are hybridized to PCR amplified target DNA or a number of different mutations when the oligonucleotides are attached to the hybridizing membrane and hybridized with labeled target DNA.

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) Nucleic Acids Res. 17:2437-2448) or at the extreme 3' end of one primer where, under appropriate conditions, mismatch can prevent, or reduce polymerase extension (Prossner (1993) Tibtech 11:238). In addition it may be desirable to introduce a novel restriction site in the region of the mutation to create cleavage-based detection (Gasparini et al. (1992) Mol. Cell Probes 6:1). It is anticipated that in certain embodiments amplification may also be performed using Taq ligase for amplification (Barany (1991) Proc. Natl. Acad. Sci USA 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing prepackaged diagnostic kits comprising at least one probe nucleic acid or antibody reagent described herein, which may be conveniently used, *e.g*., in clinical settings to diagnose subjects exhibiting symptoms or family history of a disease or illness involving a marker gene.

Furthermore, any cell type or tissue in which marker is expressed may be utilized in the prognostic assays described herein.

### 3. Monitoring of Effects During Clinical Trials

Monitoring the influence of agents (*e.g*., drugs) on the expression or activity of a marker protein (*e*.*g*., the modulation of type I diabetes can be applied not only in basic drug screening, but also in clinical trials. For example, the effectiveness of an agent determined by a screening assay as described herein to increase marker gene expression, protein levels, or upregulate marker activity, can be monitored in clinical trials of subjects exhibiting decreased marker gene expression, protein levels, or downregulated marker activity. Alternatively, the effectiveness of an agent determined by a screening assay to decrease marker gene expression, protein levels, or downregulate marker activity, can be monitored in clinical trials of subjects exhibiting increased marker gene expression, protein levels, or upregulated marker activity. In such clinical trials, the expression or activity of a marker gene, and preferably, other genes that have been implicated in, for example, a marker-associated disorder (*e.g.,* type I diabetes can be used as a "read out" or markers of the phenotype of a particular cell.

For example, and not by way of limitation, genes, including marker genes and genes encoding a marker protein disclosed that are modulated in cells by treatment with an agent (*e.g.,* compound, drug or small molecule) which modulates marker activity (e.g., identified in a screening assay as described herein) can be identified. Thus, to study the effect of agents on marker-associated disorders (*e*.*g*., type I diabetes, for example, in a clinical trial, cells can be isolated and RNA prepared and analyzed for the levels of expression of marker and other genes implicated in the marker-associated disorder, respectively. The levels of gene expression (*e.g*., a gene expression pattern) can be quantified by northern blot analysis or RT-PCR, as described herein, or alternatively by measuring the amount of protein produced, by one of the methods as described herein, or by measuring the levels of activity of marker or other genes. In this way, the gene expression pattern can serve as a marker, indicative of the physiological response of the cells to the agent. Accordingly, this response state may be determined before, and at various points during treatment of the individual with the agent.

In a preferred embodiment, a method is provided for monitoring the effectiveness of treatment of a subject with an agent (*e*.*g* , an agonist, antagonist, peptidomimetic, protein, peptide, nucleic acid, small molecule, or other drug candidate identified by the screening assays described herein) including the steps of (i) obtaining a pre-administration sample from a subject prior to administration of the agent; (ii) detecting the level of expression of a marker protein, mRNA, or genomic DNA in the preadministration sample; (iii) obtaining one or more post-administration samples from the subject; (iv) detecting the level of expression or activity of the marker protein, mRNA, or genomic DNA in the post-administration samples; (v) comparing the level of expression or activity of the marker protein, mRNA, or genomic DNA in the pre-administration sample with the marker protein, mRNA, or genomic DNA in the post administration sample or samples; and (vi) altering the administration of the agent to the subject accordingly. For example, increased administration of the agent may be desirable to increase the expression or activity of marker to higher levels than detected, *i.e.,* to increase the effectiveness of the agent. Alternatively, decreased administration of the agent may be desirable to decrease expression or activity of marker to lower levels than detected, *i*.*e*. to decrease the effectiveness of the agent. According to such an embodiment, marker expression or activity may be used as an indicator of the effectiveness of an agent, even in the absence of an observable phenotypic response.

### Pharmacogenomics

The marker protein and nucleic-acid molecules disclosed, as well as agents, or modulators which have a stimulatory or inhibitory effect on marker protein activity (*e.g*., marker gene expression) as identified by a screening assay described herein can be administered to individuals to treat (prophylactically or therapeutically) marker-associated disorders (*e*.*g*., type I diabetes or an NKT-associated condition) associated with aberrant marker protein activity. In conjunction with such treatment, pharmacogenomics (*i.e.,* the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a marker molecule or marker modulator as well as tailoring me dosage and/or therapeutic regimen of treatment with a marker molecule or marker modulator.

Pharmacogenomics deals with clinically significant hereditary variations in the response to drugs due to altered drug disposition and abnormal action in affected persons. See, for example, Eichelbaum, M. et al. (1996) Clin. Exp. Pharmacol. Physiol. 23(10-11):983-985 and Linder, M.W. et al. (1997) Clin. Chem. 43(2):254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (*e.g*., a "biallelic" gene marker map which consists of 60,000-100,000 polymorphic or variable sites on the human genome, each of which has two variants.) Such a high-resolution genetic map can be compared to a map of the genome of each of a statistically significant number of subjects taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach", can be utilized to identify genes that predict drug response. According to this method, if a gene that encodes a drugs target is known (*e.g*., a marker protein disclosed), all common variants of that gene can be fairly easily identified in the population and it can be determined if having one version of the gene versus another is associated with a particular drug response.

As an illustrative embodiment, the activity of drug metabolizing enzymes is a major determinant of both the intensity and duration of drug action. The discovery of genetic polymorphisms of drug metabolizing enzymes (*e.g*., N-acetyltransferase 2 (NAT 2) and cytochrome P450 enzymes CYP2D6 and CYP2C19) has provided an explanation as to why some subjects do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. The other extreme are the so called ultra-rapid metabolizers who do not respond to standard doses. Recently, the molecular basis of ultra-rapid metabolism has been identified to be due to CYP2D6 gene amplification.

Alternatively, a method termed the "gene expression profiling", can be utilized to identify genes that predict drug response. For example, the gene expression of an animal dosed with a drug (*e*.*g*., a marker molecule or marker modulator disclosed can give an indication whether gene pathways related to toxicity have been turned on.

Information generated from more than one of the above pharmacogenomics approaches can be used to determine appropriate dosage and treatment regimens for prophylactic or therapeutic treatment an individual. This knowledge, when applied to dosing or drug selection, can avoid adverse reactions or therapeutic failure and thus enhance therapeutic or prophylactic efficiency when treating a subject with a marker molecule or marker modulator,such qs a modulator identified by one of the exemplary screening assays described herein.

This invention is further illustrated by the following examples which should not be construed as limiting.

### EXAMPLES

### EXAMPLE 1: INHIBITOR STUDIES

A known difference between NKT cells in diabetic versus nondiabetic subjects is that while both cells are known to secrete IFN-γ upon activation, the diabetic NKT cells do not secrete IL-4 upon activation, whereas the nondiabetic NKT cells do. To identify which of the known signaling cascades initiated by T antigen receptor ligation played a dominant role in IL-4 secretion, a series of inhibitor studies was performed. *(*Wilson et al. (2000) PNAS 97:7411-7416).

Single, Vα24-positive, CD4/8 negative single-cell sorts were grown on irradiated allogeneic feeders at 50,000 cells per well with 5,000 cells per well irradiated (5,000 rads) 721.221 lymphoblastoid cells with 1 µg/ml PHA-P, IL-2 and IL-7 each at 10 units/ml (Boehringer Mannheim) and propagated as described (Wilson et al. (1998) Nature 391: 177-181). Clones positive for Vα24 and NKR-P1A by flow cytometry and a Vα24JαQ CDR3 T cell antigen receptor sequence were assayed for cytokine secretion in C1R/CD1s restriction experiments. For cytokine secretion and inhibitor studies, Vα24JαQ T cell clones GW4 (nondiabetic) and ME10 (diabetic) at 5 x 10⁴ cells per well were activated with plate-bound anti-CD3 or Ig control at 1 µg/ml. Secreted IL-4 and IFN-γ were assayed by ELISA after 4 h of activation as described *(*Wilson et al. (1998) Nature 391: 177-181). Optimal concentrations of inhibitors previously were determined by inhibitor dose-response experiments. The concentrations of inhibitors used were 10 nM wortmannin; 10 µM LY294002, 50 µM PD98059, a mitogen-activated protein kinase kinase inhibitor, and 50 µM SB203580, a p38 kinase inhibitor. The concentrations of phorbol ester and calcium ionophore used were 1 ng/ml phorbol 12-myristate 13-acetate (PMA) and 1 µg/ml ionomycin. Cyclosporin A (CsA) was used at 5 ng/ml as a negative control, due to its ability to prevent T cell activation. Calcium flux was determined by loading cells with indo-1 as per the manufacturer's specifications (Molecular Probes), followed by activation with anti-CD3 at 10 µg/ml. Maximal calcium flux was determined by the addition of ionomycin.

Both phosphoinositide-3-OH kinase (PI3 kinase) inhibitors wortmannin and LY294002 blocked anti-CD3-induced IL-4 secretion from the IL-4⁺ clone, but had no effect on the secretion of IFN-γ from either the IL-4⁺ or IL-4-null clones (Figure 2). In contrast, inhibition of the MEK kinase by PD98059 or the JNK and p38 cascades with SB203580 (Rincon and Flavell (1997) Curr. Biol. 7(11):R729-32; Dumont et al. (1998) J. Immunol. 160(6):2579-89) had no effect. After inhibition of PI3-kinase, IL-4 secretion could be rescued in the IL-4⁺ clone by the inclusion of the phorbol ester PMA or the calcium ionophore ionomycin. Neither of these substances alone or in combination repaired the defect in IL-4 secretion from the diabetic-derived clone ME10. In addition, Vα24JαQ T cell clones derived from diabetic individuals had a diminished capacity to accumulate intracellular calcium after anti-CD3 stimulation (Figure 2). These data suggest that the observed discordant IL-4 phenotype seen after T cell antigen receptor ligation cannot simply be located upstream of PI3-kinase, and that multiple genetic/regulatory differences are likely involved, including differences in proteins that regulate calcium flux.

### EXAMPLE 2: IDENTIFICATION AND CHARACTERIZATION OF MARKER cDNA

### A. Flow cytometry

Peripheral blood leukocytes were stained with fluorescently-conjugated monoclonal antibodies. Stained cells were analyzed on FACScan cytometer (Beckton Dickinson) and single-cell sorting and calcium flux determinations were performed by using a MoFlo cytometer (Cytomation, Fort Collins, NJ) as described (Wilson et al. (1998) Nature 391: 177-181).

### B. Cell culture

Single, Vα24-positive, CD4/8 negative single-cell sorts were grown on irradiated allogeneic feeders at 50,000 cells per well with 5,000 cells per well irradiated (5,000 rads) 721.221 lymphoblastoid cells with 1 µg/ml PHA-P, IL-2 and IL-7 each at 10 units/ml (Boehringer Mannheim) and propagated as described (Wilson et al. (1998) Nature 391: 177-181).

### C. Messenger RNA expression

Vα24JαQ T cell clones GW4 and ME10 (1 x 10⁷ cells) were activated for 4 h with 10 µg/ml soluble anti-CD3 or control IgG. The 4 h time point was selected due to it having been used in a previous analysis of cytokine secretion in clones derived from monozygotic twin pairs discordant for type 1 diabetes *(*Wilson et al. (1998) Nature 391(6663):177-81). Optimal concentrations of anti-CD3 previously were determined by dose-response experiments measuring cytokine secretion. Total RNA was isolated with Qiagen Rneasy kits. Total RNA then was converted to double-stranded cDNA by priming with an oligo (dT) primer that included a T7 RNA polymerase promoter site at the 5' end. The cDNA was used directly in an *in vitro* transcription reaction in the presence of biotinylated nucleotides to produce labeled cRNA (antisense RNA), which was hybridized overnight to Genechips (Affymetrix, San Jose, CA). After staining with phycoerythrin-streptavidin, the fluorescence of bound RNA was quantitated by using a GeneChip Reader (a modified confocal microscope; Affymetrix), using standard protocols.

### D. Data Analysis

The number of genes with detectable expression either before or after stimulation was nearly identical for the IL-4 null and IL-4-secreting clones (1,523 and 1,558, respectively). The frequency of expression of the majority of transcripts was unchanged by activation. The number of genes whose expression after anti-CD3 stimulation was found to increase or decrease by at least 2-fold relative to unstimulated genes were 86 (6%) and 226 (15%) in the IL-4-null and IL-4+ clones, respectively.

To more thoroughly analyze the differences in gene expression between the IL-4-null and IL-4-secreting clones, genes were grouped into six distinct expression patterns, by using the Self-Organizing Map algorithm (Figure 3) (Tamayo et al. (1999) Proc. Natl. Acad Sci. USA 96: 2907-2912). All genes modulated at least 2-fold on anti-CD3 stimulation in either the IL-4-secreting or IL-4-null clones were clustered according to the relative behavior of each gene in the two clones. The first panel of Figure 3 displays the results for all genes meeting the 2-fold criterion, and the other 11 panels show the results for specific functional classes. The dominant pattern that emerged is represented in row 1, column 2 and contains genes that were up-regulated upon activation in the IL-4-secreting clone but that were nonresponsive to stimulation in the IL-4 null clone. This finding was true for all functional classes examined, indicating a profound defect in transcriptional induction for a large number of genes in the IL-4-null clone. However, examination of the other five clusters revealed that the transcriptional dysregulation in the IL-4-null clone is more complex than merely a global nonresponsiveness, as evidenced by a group of genes that were induced in this clone but not in the IL-4-secreting clone (row 1, column 1 (Table 1)) and by a group that contained genes that were down-regulated in the IL-4-null clone but up-regulated in the IL-4-secreting clone (row 2, column 2 (Table 5)). The IL-4-null clone is, therefore, able to respond to stimulation through the T cell receptor.

Six different expression patterns were observed. Table 1 (representative of row 1, column 1 in each of the clusters set forth in Figure 3) lists each of the genes which were observed to be increased in expression in activated diabetic NKT cells and unchanged or increasing to a lesser extent in expression in activated nondiabetic NKT cells, relative to appropriate resting control cells. Table 2 (representative of row 1, column 2 in each of the clusters set forth in Figure 3) lists each of the genes which were observed to be unchanged in expression in activated diabetic NKT cells relative to control resting cells, but which are increased in expression in activated nondiabetic NKT cells relative to resting control cells. Table 3 (representative of row 1, column 3 in each of the clusters set forth in Figure 3) lists each of the genes which were observed to be increased in expression in both activated diabetic and nondiabetic NKT cells relative to appropriate resting control cells. Table 4 (representative of row 2, column 1 in each of the clusters set forth in Figure 3) lists those genes which were observed to be decreased in expression in activated nondiabetic NKT cells relative to resting control cells, but which were unchanged in expression in activated diabetic NKT cells relative to resting control cells. Table 5 (representative of row 2, column 2 in each of the clusters set forth in Figure 3) lists those genes which were observed to be increased in expression in activated nondiabetic NKT cells relative to resting control cells, but which were decreased in expression in activated diabetic NKT cells relative to resting control cells. Table 6 (representative of row 2, column 3 in each of the clusters set forth in Figure 3) lists those genes which were observed to be decreased in expression in activated diabetic NKT cells relative to resting control cells, but which were unchanged or decreasing to a lesser extent in expression in nondiabetic NKT cells relative to resting control cells.

Significant changes in transcription of members of the cytokine/chemokine family between the IL-4+ and IL-4-null clones were observed, and were confirmed at the protein level by ELISA assay. Significant expression differences were also detected in other genes important for cell survival, cytokine secretion, and calcium flux that in part are activated through PI3-kinase signaling, such as BCLxL, IAP, PLCgammal and the tec family kinase, Itk, transcripts for which were found in greater abundance in the IL-4+ clone. Differences were also observed in the expression of mRNAs encoding transcription factors and signaling modulators important for cytokine secretion and Th phenotype, including GATA3, STAT1, STAT4, JunB, JunD, and NFAT4. Certain of these genes were increased in expression in the IL-4+ clone (GATA3, JunB and JunD), while the remainder were increased in expression in the IL-4-null clone.

### EXAMPLE 3: Gene Expression In NKT, CD4, and CD8 T Cell Clones

### A. Approach

NKT, CD4, and CD8 T cell clones were generated from a single donor using the methods described above. A single clone of each type was selected and stimulated with anti-CD3 for 2, 4, 8, 24 or 48 hours to create a kinetic activation series. Three replicate experiments were performed for CD4 and CD8, but only one replicate was performed for NKT. The query for the NKT replicate imposed a three-fold change filter. No change filter was imposed for CD4 and CD8; rather, the filter imposed was that genes had to increase or decrease relative to 0 hour for all three replicates. RNA was isolated and analyzed on chips which monitored 12,000 known human genes. The expression data was queried for genes with consistent expression patterns among the three repetitions, and with three-fold changes between the different T cell subsets.

### B. Results

Genes which were identified in a query requiring at least a three-fold increase in mRNA levels in at least one time point in the NKT cell sample, and had an expression pattern or magnitude different from CD4 and CD8 cell samples are set forth in Table 9. Genes which were identified in a query requiring an increase in mRNA levels in at least one time point for all three replications of the experiment in CD4 cell samples, and had an expression pattern or magnitude different from NKT and CD8 cell samples are set forth in Table 10. Genes set forth in Table 9 were excluded from Table 10. Genes which were identified in a query requiring at an increase in mRNA levels in at least one time point for all three replications of the experiment in CD8 cell samples, and had an expression pattern or magnitude different from NKT or CD4 cell samples, are set forth in Table 11. Genes set forth in Tables 9 and 10 were excluded from Table 11.

### EXAMPLE 4: Gene Expression In Unstimulated NKT, CD4, and CD8 T Cell Clones

### A. Approach

NKT, CD4, and CD8 T cell clones were generated from a single donor using the methods described above. A single clone of each type was selected and differences in gene expression was observed in resting cells. Three replicate experiments were performed for CD4 and CD8, and two replicates were performed for NKT. RNA was isolated and analyzed on chips which monitored 12,000 known human genes. The expression data was queried for genes with consistent expression patterns among the three repetitions, and with three-fold changes between the different T cell subsets.

### B. Results

Genes which were identified in a query requiring a change in mRNA levels for all three replications of the experiment in resting CD4 cell samples, and had an expression pattern or magnitude different from resting NKT cell samples are set forth in Table 12. Genes which were identified in a query requiring a change in mRNA levels for all three replications of the experiment in resting CD8 cell samples, and had an expression pattern or magnitude different from resting NKT cell samples, are set forth in Table 13.

**Table 1**

| | | |
|---|---|---|
| Cytokine | Kinase/phosphatase | Signal Transduction |
| U89922 LT-β (1,1) | X79510 PTP D1 (1,1) | HT5108 TRAP-3 (1,1) |
| | | X80200 MLN62 (1,1) |
| Surface Receptor | RNA Metabolism | |
| U38276 Semaphorin III (1,1) | D38251 RNP B5 (1,1) | |
| U82169 Frizzled (1,1) | U90547 RNP homolog (1,1) | |
| | | |
| Cytoskeleton | | |
| U80184 Flightless I hom. (1,1) | | |
| | | |
| Nuclear Protein | | |
| U73477 Nuclear pp32 (1,1) | | |

**Table 2**

| | | |
|---|---|---|
| Cytokine | Kinase/phosphatase | Transcription Factor |
| J00219 IFN-γ (1,2) | L10717 ITK (1,2) | M69043 IκBα (1,2) |
| V00536 IFN-γ (1,2) | X60673 AK3 (1,2) | X58072 GATA-3 (1,2) |
| M13207 GM-CSF (1,2) | X85545 PKX-1 (1,2) | U43185 STAT-5A (1,2) |
| M16441 TNF-α (1,2) | D13720 LYK (1,2) | X51345 Jun-B (1,2) |
| X02910 TNF-α(1,2) | HT1153 Nm23-H2S | X56681 Jun-D (1,2) |
| X04688 IL-5 (1,2) | (1,2) | U15460B-ATF(1,2) |
| U31120 IL-13 (1,2) - | M30448 CK II β (1,2) | HT4899 C-myc (1,2) |
| M37435 M-CSF (1,2) | M90299 Glucokinase | L00058 C-myc (1,2) |
| U02020 PBEF (1,2) | (1,2) | M13929 C-myc (1,2) |
| U37518 TRAIL (1,2) | U08316 ISPK- (1,2) | U26173 NF-IL3A (1,2) |
| U46461 Dishevelled hom. | X80910 PPP1CB (1,2) | M97796 Id-2 (1,2) |
| (1,2) | X93920 DUSP-6 (1,2) | M96843 Id-2B (1,2) |
| | | D14826 CREM (1,2) |
| Surface Receptor | RNA Metabolism | S68271 CREM (1,2) |
| M32315 TNF-R (1,2) | X17567 RNP B (1,2) | J03827 Y Box BP (1,2) |
| U03397 4-1BB (1,2) | M29064 RNP B1 (1,2) | U09412 ZNF134 (1,2) |
| S77812 VEGF-R (1,2) | HT110 RNP A/B (1,2) | U13044 NRF-2α (1,2) |
| X01057 IL-2Rα (1,2) | Z23064 RNP G (1,2) | U22431 HIF-1α (1,2) |
| Y00285 IGF-R II (1,2) | HT3238 RNP K (1,2) | X78925 HZF-2 (1,2) |
| IGF-R (1,2) | X52979 RNP SmB (1,2) | Z47727 RNA POL2K (1,2) |
| L08096 CD27 (1,2) | U15009 RNP SmD3 | |
| Z30426 CD69 (1,2) | (1,2) | Signal Transduction |
| U76764 CD97 (1,2) | X85372 RNP Sm F (1,2) | U20158SLP-76(1,2) |
| U60800 CD100 (1,2) | U30827 SF SRp40 (1,2) | U26710 Cbl-b (1,2) |
| M24283 Rhinovirus-R (1,2) | X70944 SF (PTP-assoc.) | D78132 RHEB (1,2) |
| U19906 Arg. Vasopressin-R | (1,2) | M63573 SCYLP (1,2) |
| (1,2) | M60858 Nucleolin (1,2) | M75099 FK506 BP (1,2) |
| Z48042 P137 (1,2) | U10323 NF45 (1,2) | Z35227 TTF(1,2) |
| | U38846 Stim. of TAR | |
| Cytoskeleton | (1,2) | Protein Metabolism |
| X00351 β-Actin (1,2) | X59417 PROS-27 (1,2) | D28473 ILE-tRNA Synth. |
| U20582 Actin-like pep. (1,2) | X59892 IFN-Ind. γ2 (1,2) | (1,2) |
| X82207 β-centractin (1,2) | X66899 EWS (1,2) | U09510 GLY-tRNA Synth. |
| X98534 VASP (1,2) | X71428 fus (1,2) | (1,2) |
| | X72727 Tunp (1,2) | L25085 Sec61-β (1,2) |
| Nuclear Protein | X75755 PR264 (1,2) | -X74801 Chaperonin Cctg (1,2) |
| U62962 Int-6 (1,2) | Z24724 Poly A site (1,2) | X77584 Thioredoxin (1,2) |
| | | Y00281 Ribophorin I (1,2) |
| | Chemokine | |
| | M23178 MIP-1α (1,2) | Apoptosis |
| | J04130 MIP-1β (1,2) | Z23115 Bcl-X_{L} (1,2) |
| | M69203 MCP-1 (1,2) | U45878 IAP-1 (1,2) |
| | | U11821 Fas Ligand (1,2) |
| | | S81914IEX-1(1,2) |
| | | U37546 MIHC (1,2) |

**Table 3**

| | | |
|---|---|---|
| Surface Receptor | Kinase/phosphatase | Transcription Factor |
| D79206 Ryudocan (1,3) | U24152 PAK-1 (1,3) | J04076 EGR-2 (1,3) |
| HT3125 CD44 (1,3) | D11327 PTPN7 (1,3) | D61380 DJ-1 (1,3) |
| | U15932 DUSP-5 (1,3) | HT4567 PC4 (1,3) |
| Nuclear Protein | | |
| L25931 Lamin B Rec. (1,3) | | Signal Transduction |
| | | U19261 EBV-Ind. (1,3) |
| | RNA Metabolism | |
| | L28010 RNP F (1,3) | |
| | HT4788 RNP I (1,3) | Protein Metabolism |
| | L03532 M4 (1,3) | Y10807 ARG-methyltrans. (1,3) ARG-methyltrans. |
| | | |
| | | D13748 EIF-4AI (1,3) |
| | Chemokine | |
| | L19686 MIF (1,3) | |

**Table 4**

| | | |
|---|---|---|
| Surface Receptor | Kinase/phosphatase | Transcription Factor |
| L3 9064 IL-9R (2,1) | L16862 GRK-6 (2,1) | HT4921 BTF-3 hom. (2,1) |
| X14046 CD37 (2,1) | L27071 TXK (2,1) | |
| L31584 EBI-1 (2,1) | | Protein Metabolism |
| X97267 LPAP (2,1) | | X55733 EIF-4B (2,1) |
| | | |
| Cytoskeleton | | |
| D83735 Calponin (2,1) | | |

**Table 5**

| | | |
|---|---|---|
| Surface Receptor | Kinase/phosphatase | Signal Transduction |
| M33680 TAP-1 (2,2) | J03805 PPP2CB (2,2) | M28209 RAB-1 (2,2) |
| M63175 AMFR (2,2) | | |
| U60975 gp250 (2,2) | | |
| Z50022 C21 orf3 (2,2) | | |

**Table 6**

| | | |
|---|---|---|
| Cytokine | Kinase/phosphatase | Transcription Factor |
| M90391 IL-16 (2,3) | HT3678 CLK-1 (2,3) | L41067 NFAT-4C (2,3) |
| | U66464 HPK-1 (2,3) | L78440 STAT-4 (2,3) |
| Surface Receptor | X62535 DAG Kinase (2,3) | M82882 ELF-1 (2,3) |
| U90546 Butyrophilin BT4 | M31724 PTP-1B (2,3) | M83667 NF-IL6 (2,3) |
| (2,3) | | |
| U90552 Butyrophilin BT5 | RNA Metabolism | Signal Transduction |
| (2,3) | U69546 RNA BP (2,3) | D78577 14-3-3-Eta (2,3) |
| X96719 AICL (2,3) | | X89399 Ins(1345)P4 BP (2,3) |
| | | |
| Cytoskeleton | | Protein Metabolism |
| J00314 β-tubulin (2,3) | | X76648 Glutaredoxin (2,3) |
| M21812 Myosin LC (2,3) | | |
| X98411 Myosin-IE (2,3) | | |
| | | |
| Nuclear Protein | | |
| M17733 Thymosin-β4 | | |
| (2,3) | | |

**Table 7**

| | | |
|---|---|---|
| Accession Number | Name of Gene | Citation |
| | PGE-2 synthase | Litherland *et al.* (1999) *J*. |
| | | *Clin. Invest.* 104: 515-523 |
| | | |
| NM 005191 | CD80 | Takahashi *et al.* (1998) *J*. |
| NM 006889 | CD86 | *Immunol.* 161: 2629-2635 |
| AF 142665 | CD1a | |
| | | |
| NM 005214 | CTLA4 | |

**TABLE 8**

| | | |
|---|---|---|
| | (I38700) NKR-P1A | |
| | (NP 009330) STAT1 | |

**Table 9**

| Accession Number | Gene |
|---|---|
| J00219 | IFNG |
| M11717 | HSP A1A |
| L05424 | CD44 |
| X51757 | HSP A6 |
| M28130 | IL-8 |
| X00695 | IL-2 |
| J00219 | IFNG |
| U61836 | FLJ20746 |
| M59040 | CD44 |
| L19779 | H2 AFO |
| D63789 | SCYC2 |
| M59830 | HSP A1B |
| AI885852 | H2 AFO |
| M91196 | ICS BP1 |
| AF008915 | EVI5 |
| X81851 | IL-4 |
| S82692 | IL-2 |
| X71661 | LMAN1 |
| M27533 | CD80 |
| M17017 | IL-8 |
| X51757 | HSP A6 |
| U10550 | GEM |
| AF079221 | BNIP 3L |
| AF060568 | ZNF145 |
| W29115 | FLJ20500 |
| D11466 | PIGA |
| X51956 | ENO2 |
| U84371 | AK2 |
| M73255 | VCAM1 |
| U48730 | STAT 5B |
| U31120 | IL-13 |
| U43185 | STAY 5A |
| M30257 | VCAM1 |
| L31584 | CCR7 |
| AJ001684 | KLRC2 |

**Table 10**

| Accession Number | Gene |
|---|---|
| U16720 | IL-10 |
| AF002668 | DEGS |
| M97936 | STAT1 |
| X76220 | MAL |
| X04430 | IL-6 |
| M69199 | GOS2 |
| D86324 | CMAH |
| AF015287 | SPUVE |
| U64197 | SCYA20 |
| L05072 | IRF1 |
| U83171 | SCYA22 |

**Table 11**

| Accession Number | Gene |
|---|---|
| J04988 | HSP CB |
| U12595 | TRAP1 |
| L77886 | PTPRK |
| X17620 | NME1 |
| U24152 | PAK |
| U24152 | PAK1 |
| U43899 | STAM |
| X03541 | NTRK1 |
| X55504 | NOL1 |
| X73066 | NME1 |
| M64231 | SRM |
| AC005546 | UNK_{A}C005546 |
| Y10805 | HRM1L2 |
| AB011083 | ADCY3 |
| W28612 | UNK_{W}28612 |
| D13626 | KIAA0001 |
| X15306 | NEFH |
| Y13834 | ZMPSTE24 |
| M57506 | SCYA1 |
| D31887 | KIAA0062 |
| M16660 | HSP CB |
| U59151 | DKC 1 |
| Y12065 | NOP56 |
| AF059531 | PRMT3 |
| AL050205 | UNK_{A}L050205 |
| U78525 | EIF3S9 |
| D50914 | KIAA0124 |
| AJ001014 | RAMP1 |
| AB024301 | RUVBL2 |
| AF026166 | CCT2 |
| AB028965 | KIAA1042 |
| D78130 | SQLE |
| Y18643 | METTL1 |
| U86602 | P40 |
| U13737 | CASP3 |
| U23143 | SHMT2 |
| AF008442 | RPA40 |
| S85655 | PHB |
| D21262 | P130 |
| L33842 | IMPDH2 |
| X03541 | NTRK 1 |
| D25218 | KIAA0112 |
| AB002359 | PFAS |
| AL049422 | UNK_{A}L049422 |
| U94317 | RPP40 |
| X95263 | PWP2H |
| M69039 | C1QBP |
| U16799 | ATP1B1 |
| M223 82 | HSPD1 |
| X06323 | MRPL3 |
| X80200 | TRAF4 |
| D26488 | KIAA00007 |
| X54199 | GART |
| AL050159 | DKFZP586A0522 |
| D82348 | ATIC |
| AL038662 | NME1 |
| AI912041 | HSPE1 |
| M38690 | CD9 |
| AJ007398 | DKFZP564M182 |
| D87432 | SLC7A6 |
| W52003 | KIAA1237 |
| U31382 | . GANG4 |
| M31516 | DAF |
| AB014547 | MTMR4 |
| D43950 | CCT5 |
| AL080119 | PAI-RBP1 |
| AL080119 | PAI-RBP1 |
| U75686 | PABPC4 |
| AF081280 | NPM3 |
| X74801 | CCT3 |
| U04953 | IARS |
| AB018293 | KIAA0750 |
| U51166 | TDG |
| AA926957 | FLJ10534 |
| AL050022 | DKFZP564D116 |
| AA772359 | PSMA2 |
| AI553745 | HSPC111 |
| AI816034 | NOLA2 |
| L36720 | BYSL |
| U28042 | DDX10 |
| Y10256 | MAP3K14 |

**Table 12**

| Accession Number | Gene | CD4 Levels Relative To NKT Levels |
|---|---|---|
| M21121 | SCYA5 | decrease |
| J04765 | SPP1 | increase |
| AE000659 | UNKAE | decrease |
| D63789 | SCYC2 | decrease |
| M57703 | PMCH | increase |
| A001685 | KLRC3 | decrease |
| AJ001684 | KLRC2 | decrease |
| M57888 | GZMB | decrease |
| M28393 | PRF1 | decrease |
| AF052124 | SPP1 | increase |
| U11276 | KLRB1 | decrease |
| AF004230 | LILRB1 | increase |
| D90144 | SCYA3 | decrease |
| 575168 | MATK | decrease |
| S69115 | NKG7 | decrease |
| AB013924 | TSC403 | increase |
| Z22576 | CD69 | decrease |
| AF031137 | D6S49E | decrease |
| W60864 | TYROBP | decrease |
| AL031983 | GABBR1 | increase |
| AF013611 | CTSW | decrease |
| AI651806 | CRIM1 | decrease |
| AB023209 | KIAA0992 | increase |
| M30894 | TRGα | decrease |
| J04430 | ACP5 | increase |
| L08177 | EB12 | increase |
| S78187 | CDC25B | increase |
| X767711 | FEN1 | increase |
| L25876 | CDKN3 | increase |
| U73379 | UBCH10 | increase |
| M31303 | LAP18 | increase |
| X51688 | CCNA2 | increase |
| X61079 | UNK X61 | increase |
| X63547 | USP6 | increase |
| 568134 | CREM | decrease |
| AF015254 | STK12 | increase |
| Y14768 | UNK Y14 | increase |
| M25753 | CCNB1 | increase |
| D 14678 | KNSL2 | increase |
| AB017430 | KNSL4 | increase |
| AF067656 | ZWINT | increase |
| D90144 | SCYA3 | decrease |
| U63743 | KNSL6 | increase |
| AB000115 | GS3686 | decrease |
| U30872 | CENPF | increase |
| D14657 | KIAA0101 | increase |
| D79987 | KIAA0165 | increase |
| W60864 | TYROBP | decrease |
| U05340 | CDC20 | increase |
| M63928 | TNFRSF7 | increase |
| X13444 | CD8B1 | increase |
| X16665 | HOXB2 | decrease |
| X14850 | H2AFX | increase |
| X70944 | SFPQ | increase |
| U37426 | KNSL1 | increase |
| M30894 | TRGα | decrease |
| X65550 | MKI67 | increase |
| M86699 | ZU | increase |
| L47276 | UNK L47 | increase |
| X16302 | IGFBP2 | increase |
| AA100961 | PECAM1 | increase |
| M94345 | CAPG | increase |
| V00599 | TUBS | increase |
| AE000659 | UNK AE | decrease |
| AJ001684 | KLRC2 | decrease |
| D86096 | UNK D86 | increase |
| AF053306 | BUB1B | increase |
| AF032862 | HMMR | increase |
| AI375913 | TOP2A | increase |
| AL080146 | CCNB2 | increase |

**Table 13**

| | | |
|---|---|---|
| M25753 | CCNB1 | increase |
| D11139 | TIMP1 | increase |
| J04088 | TOP2A | increase |
| X65550 | MKI67 | increase |
| X68742 | ITGA1 | increase |
| D26361 | KIAA0042 | increase |
| AJ001685 | KLRC3 | increase |
| M57888 | GZMB | decrease |
| X68742 | ITGA1 | increase |
| D88357 | CDC2 | increase |
| AC004142 | FLJ11129 | increase |
| AB023209 | KIAA0992 | increase |
| U16954 | AF1 Q | increase |
| AB024704 | C20ORF1 | increase |
| X05360 | CDC2 | increase |
| D00596 | TYMS | increase |
| M12824 | CD8A | increase |
| U74612 | FOXM1 | increase |
| AA926959 | CKS1 | increase |
| AA22530 | FLJ20500 | decrease |
| U14518 | CENPA | increase |

## Claims

1. A method of assessing whether a subject is afflicted with type I diabetes, the method comprising comparing:
a) the level of expression of a marker in a sample of NKT cells from a subject, wherein the marker is selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, and
b) the normal level of expression of the marker in a control sample of NKT cells, wherein a significant difference between the level of expression of the marker in the sample of NKT cells from the subject and the normal level is an indication that the subject is afflicted with type I diabetes.

2. The method of claim 1, wherein the marker corresponds to a transcribed polynucleotide or portion thereof, wherein the polynucleotide comprises the marker.

3. The method of claim 1, wherein the NKT cells are collected from pancreatic tissue.

4. The method of claim 1, wherein the NKT cells are collected from blood tissue.

5. The method of claim 1, wherein the level of expression of the marker in the sample of NKT cells differs from the normal level of expression of the marker in a subject not afflicted with type I diabetes, by a factor of at least 2.

6. The method of claim 1, wherein the level of expression of the marker in the sample differs from the normal level of expression of the marker in a subject not afflicted with type I diabetes, by a factor of at least 5.

7. The method of claim 1, wherein the level of expression of the marker in the sample is assessed by detecting the presence in the sample of a protein corresponding to the marker.

8. The method of claim 7, wherein the presence of the protein is detected using a reagent which specifically binds with the protein.

9. The method of claim 8, wherein the reagent is selected from the group consisting of an antibody, an antibody derivative, and an antibody fragment.

10. The method of claim 1, wherein the level of expression of the marker in the sample is assessed by detecting the presence in the sample of a transcribed polynucleotide or portion thereof, wherein the transcribed polynucleotide comprises the marker.

11. The method of claim 10, wherein the transcribed polynucleotide is an mRNA.

12. The method of claim 10, wherein the transcribed polynucleotide is a cDNA.

13. The method of claim 10, wherein the step of detecting further comprises amplifying the transcribed polynucleotide.

14. The method of claim 1, wherein the level of expression of the marker in the sample is assessed by detecting the presence in the sample of a transcribed polynucleotide which anneals with the marker or anneals with a portion of a polynucleotide, wherein the polynucleotide comprises the marker, under stringent hybridization conditions.

15. The method of claim 1, comprising comparing:
a) the level of expression in the sample of NKT cells of each of a plurality of markers independently selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, and
b) the normal level of expression of each of the plurality of markers in samples of the same type obtained from control subjects not afflicted with type I diabetes, wherein the level of expression of more than one of the markers is significantly altered, relative to the corresponding normal levels of expression of the markers, indicating that the subject is afflicted with type I diabetes.

16. The method of claim 15, wherein the plurality comprises two or more of the markers.

17. The method of claim 15, wherein the plurality comprises at least five of the markers.

18. A method for monitoring the progression of type I diabetes in a subject, the method comprising:
a) detecting in a subject sample of NKT cells at a first point in time, the expression of a marker, wherein the marker is selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, and combinations thereof;
b) repeating step a) at a subsequent point in time; and c) comparing the level of expression detected in steps a) and b), and therefrom monitoring the progression of type I diabetes in the subject.

19. The method of claim 18, wherein the marker corresponds to a transcribed polynucleotide or portion thereof, wherein the polynucleotide comprises the marker.

20. The method of claim 18, wherein the NKT cells are collected from pancreatic tissue.

21. The method of claim 18, wherein the NKT cells are collected from blood tissue.

22. A method of assessing the efficacy of a therapy for inhibiting type I diabetes, the method comprising comparing:
a) expression of a marker in a first sample of NKT cells obtained from the subject prior to providing at least a portion of the therapy to the subject, wherein the marker is selected from the group consisting of
LT-P, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, and
b) expression of the marker in a second sample of NKT cells obtained from the subject following provision of the portion of the therapy, wherein a significantly lowered level of expression of the marker in the second sample, relative to the first sample, is an indication that the therapy is efficacious for inhibiting type I diabetes in the subject.

23. A method of assessing the efficacy of a test compound for inhibiting type I diabetes in a subject, the method comprising comparing:
a) expression of a marker in a first sample of NKT cells obtained from the subject and exposed to or maintained in the presence of the test compound, wherein the marker is selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, and
b) expression of the marker in a second sample of NKT cells obtained from the subject, wherein the second sample is not exposed to the test compound, wherein a significantly lower level of expression of the marker in the first sample, relative to the second sample, is an indication that the test compound is efficacious for inhibiting type I diabetes in the subject.

24. The method of claim 23, wherein the first and second samples are portions of a single sample of NKT cells obtained from the subject.

25. The method of claim 23, wherein the first and second samples are portions of pooled samples of NKT cells obtained from the subject.

26. A method of selecting a composition for inhibiting type I diabetes in a subject, the method comprising:
a) separately maintaining aliquots of a sample comprising NKT cells obtained from a subject in the presence of a plurality of test compositions;
b) comparing expression of a marker in each of the aliquots, wherein the marker is selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B; and
c) selecting one of the test compositions which induces a lower level of expression of the marker in the aliquot containing that test composition, relative to other test compositions.

27. A method of assessing the potential of a test compound to trigger type I diabetes in a cell, the method comprising:
a) maintaining separate aliquots of NKT cells in the presence and absence of the test compound; and
b) comparing expression of a marker in each of the aliquots, wherein the marker is selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B,
wherein a significantly enhanced level of expression of the marker in the aliquot maintained in the presence of the test compound, relative to the aliquot maintained in the absence of the test compound, is an indication that the test compound possesses the potential for triggering type I diabetes in a cell.

28. Use of a kit for conducting the methods according to any one of claims 1, 23, 26 and 27, wherein the kit comprises at least one reagent for assessing the expression of a marker in a sample of NKT cells, wherein the marker is selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B.

29. The use according to claim 28, wherein the reagent is a nucleic acid probe that specifically binds with a transcribed polynucleotide corresponding to a marker selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B.

30. The use according to claim 28, wherein the reagent is an antibody that specifically binds with a protein corresponding to a marker selected from the group consisting of
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B.

## Patentansprüche

1. Verfahren zur Beurteilung ob ein Proband an Diabetes vom Typ I leidet, wobei das Verfahren ein Vergleichen umfasst von:
a) dem Expressionsniveau eines Markers in einer Probe von NKT-Zellen eines Probanden, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, und
b) dem normalen Expressionsniveau des Markers in einer Kontrollprobe von NKT-Zellen, wobei ein signifikanter Unterschied zwischen dem Expressionsniveau des Markers in der Probe der NKT-Zellen aus dem Proband und dem normalen Niveau eine Anzeige ist, dass der Proband an Diabetes vom Typ I leidet.

2. Verfahren nach Anspruch 1, wobei der Marker ein transkribiertes Polynukleotid oder einem Teilbereich davon entspricht, wobei das Polynukleotid den Marker umfasst.

3. Verfahren nach Anspruch 1, wobei die NKT-Zellen aus Bauchspeicheldrüsengewebe gesammelt werden.

4. Verfahren nach Anspruch 1, wobei die NKT-Zellen aus Blutgewebe gesammelt werden.

5. Verfahren nach Anspruch 1, wobei das Expressionsniveau des Markers in der Probe der NKT-Zellen von dem normalen Expressionsniveau des Markers in einem Probanden, der nicht an Diabetes vom Typ I leidet, sich um einen Faktor von wenigstens 2 unterscheidet.

6. Verfahren nach Anspruch 1, wobei das Expressionsniveau des Markers in der Probe der NKT-Zellen von dem normalen Expressionsniveau des Markers in einem Probanden, der nicht an Diabetes vom Typ I leidet, sich um einen Faktor von wenigstens 5 unterscheidet.

7. Verfahren nach Anspruch 1, wobei das Expressionsniveau des Markers in der Probe durch Nachweis der Anwesenheit eines dem Marker entsprechenden Proteins in der Probe beurteilt wird.

8. Verfahren nach Anspruch 7, wobei die Anwesenheit des Proteins unter Verwendung eines Reagenz nachgewiesen wird, welches spezifisch an das Protein bindet.

9. Verfahren nach Anspruch 8, wobei das Reagenz ausgewählt wird aus der Gruppe bestehend aus einem Antikörper, einem Antikörperderivat und einem Antikörperfragment.

10. Verfahren nach Anspruch 1, wobei das Expressionsniveau des Markers in der Probe durch Nachweisen der Anwesenheit eines transkribierten Polynukleotids oder eines Teilbereichs davon in der Probe beurteilt wird, wobei das transkribierte Polynukleotid den Marker umfasst.

11. Verfahren nach Anspruch 10, wobei das transkribierte Polynukleotid eine mRNA ist.

12. Verfahren nach Anspruch 10, wobei das transkribierte Polynukleotid eine cDNA ist.

13. Verfahren nach Anspruch 10, wobei der Schritt des Nachweises weiterhin die Vervielfältigung des transkribierten Polynukleotids umfasst.

14. Verfahren nach Anspruch 1, wobei das Expressionsniveau des Markers in der Probe durch Nachweis der Anwesenheit eines transkribierten Polynukleotids in der Probe beurteilt wird, welches unter stringenten Hybridisierungsbedingungen an den Marker anlagert oder an einen Teilbereich eines Polynukleotids anlagert, wobei das Polynukleotid den Marker umfasst.

15. Verfahren nach Anspruch 1, umfassend ein Vergleichen von:
a) dem Expressionsniveaum von je einem einer Mehrzahl von Markern in der Probe der NKT-Zellen, die unabhängig ausgewählt werden aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, und
b) dem normalen Expressionsniveau jedes der Mehrzahl von Markern in Proben der gleichen Art erhalten von Kontrollprobanden, die nicht an Diabetes vom Typ I leiden, wobei das Expressionsniveau von mehr als einem der Marker relativ zu den entsprechenden normalen Expressionsniveaus der Marker signifikant verändert ist, was anzeigt, dass der Proband an Diabetes vom Typ I leidet.

16. Verfahren nach Anspruch 15, wobei die Mehrzahl zwei oder mehr der Marker umfasst.

17. Verfahren nach Anspruch 15, wobei die Mehrzahl wenigstens fünf der Marker umfasst.

18. Verfahren zur Überwachung der Entwicklung von Diabetes vom Typ I in einem Probanden, wobei das Verfahren umfasst:
a) Nachweisen der Expression eines Markers in einer Probandenprobe von NKT-Zellen zu einem ersten Zeitpunkt, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B und Kombinationen davon;
b) Wiederholen von Schritt a) zu einem späteren Zeitpunkt; und
c) Vergleichen des in den Schritten a) und b) nachgewiesenen Expressionsniveaus und daher Überwachen der Entwicklung der Diabetes vom Typ I in dem Probanden.

19. Verfahren nach Anspruch 18, wobei der Marker einem transkribierten Polynukleotid oder einem Teilbereich davon entspricht, wobei das Polynukleotid den Marker umfasst.

20. Verfahren nach Anspruch 18, wobei die NKT-Zellen aus Bauchspeicheldrüsengewebe gesammelt werden.

21. Verfahren nach Anspruch 18, wobei die NKT-Zellen aus Blutgewebe gesammelt werden.

22. Verfahren für die Beurteilung der Wirksamkeit einer Therapie für die Hemmung von Diabetes vom Typ I, wobei das Verfahren umfasst:
a) Expression eines Markers in einer ersten Probe von NKT-Zellen, erhalten aus dem Probanden vor Bereitstellung wenigstens eines Teils der Therapie an den Probanden, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, und
b) Expression des Markers in einer zweiten Probe von NKT-Zellen, erhalten aus dem Probanden nach Bereitstellung des Teils der Therapie, wobei ein signifikant verringertes Expressionsniveau des Markers in der zweiten Probe relativ zu der ersten Probe eine Anzeige dafür ist, dass die Therapie wirksam für die Hemmung von Typ 1 Diabetes in dem Probanden ist.

23. Verfahren für die Beurteilung der Wirksamkeit einer Testverbindung für die Hemmung von Diabetes vom Typ I in einem Probanden, wobei das Verfahren ein Vergleichen umfasst von:
a) der Expression eines Markers in einer ersten Probe von aus dem Probanden erhaltenen NKT-Zellen, die der Testverbindung ausgesetzt oder in ihrer Anwesenheit erhalten, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B, und
b) der Expression des Markers in einer zweiten Probe von aus dem Probanden erhaltenen NKT-Zellen, wobei die zweite Probe nicht der Testverbindung ausgesetzt wird, wobei ein signifikant geringeres Expressionsniveau des Markers in der ersten Probe relativ zu der zweiten Probe eine Anzeige dafür ist, dass die Testverbindung wirksam für die Hemmung von Diabetes vom Typ I in dem Probanden ist.

24. Verfahren nach Anspruch 23, wobei die erste und die zweite Probe Teile einer einzelnen Probe von aus dem Probanden erhaltenen NKT-Zellen sind.

25. Verfahren nach Anspruch 23, wobei die ersten und zweiten Proben Teile von vereinigten Proben von aus dem Probanden erhaltenen NKT-Zellen sind.

26. Verfahren für die Auswahl einer Zusammensetzung für die Hemmung von Diabetes vom Typ I in einem Probanden, wobei das Verfahren umfasst:
a) getrenntes Erhalten von Aliquots einer Probe mit aus einem Probanden erhaltenen NKT-Zellen in der Anwesenheit einer Mehrzahl von Testverbindungen;
b) Vergleich der Expression eines Markers in jedem der Aliquots, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B; und
c) Auswählen einer der Testzusammensetzungen, welche relativ zu anderen Testzusammensetzungen ein geringeres Expressionsniveau des Markers in dem Aliquot induziert, das die Testzusammensetzung beinhaltet.

27. Verfahren für die Beurteilung des Potentials einer Testverbindung um Diabetes vom Typ I in einer Zelle auszulösen, wobei das Verfahren umfasst:
a) Erhalten getrennter Aliquots von NKT-Zellen in der Anwesenheit und Abwesenheit der Testverbindung; und
b) Vergleichen der Expression eines Markers in jedem der Aliquots, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B,
wobei ein signifikant erhöhtes Expressionsniveau des Markers in dem in der Anwesenheit der Testverbindung erhaltenen Aliquot, relativ zu dem in der Abwesenheit der Testverbindung erhaltenen Aliquot, eine Anzeige dafür ist, dass die Testverbindung das Potential für die Auslösung von Diabetes vom Typ I in einer Zelle besitzt.

28. Verwendung eines Reagenziensatzes für die Ausführung der Verfahren nach einem der Ansprüche 1, 23, 26 und 27, wobei der Reagenziensatz wenigstens ein Reagenz für die Beurteilung der Expression eines Markers in einer Probe von NKT-Zellen umfasst, wobei der Marker ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B.

29. Verwendung nach Anspruch 28, wobei das Reagenz eine Nukleinsäuresonde ist, die spezifisch an ein transkribiertes Polynukleotid bindet, das einem Marker entspricht, der ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B.

30. Verwendung nach Anspruch 28, wobei das Reagenz ein Antikörper ist, der spezifisch an ein Protein bindet, das einem Marker entspricht, der ausgewählt wird aus der Gruppe bestehend aus
LT-β, Semaphorin III, Frizzled, Flightless I hom., Nuclear pp32, PTP D1, RNP B5, RNP homolog, TRAP-3, MLN62, IL-9R, CD37, EBI-1, LPAP, Calponin, GRK-6, TXK, BTF-3 hom., EIF-4B.

## Revendications

1. Procédé pour évaluer si un sujet est atteint du diabète du type I, le procédé comprenant le fait de comparer:
a) le niveau d'expression d'un marqueur dans un échantillon de cellules NKT provenant d'un sujet, où le marqueur est sélectionné du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B, et
b) le niveau normal d'expression du marqueur dans un échantillon de contrôle de cellules NKT, où une différence significative entre le niveau d'expression du marqueur dans l'échantillon de cellules NKT provenant du sujet et le niveau normal est une indication que le sujet est atteint du diabète du type 1.

2. Procédé de la revendication 1, dans lequel le marqueur correspond à un polynucléotide transcrit ou une partie de celui-ci, où le polynucléotide comprend le marqueur.

3. Procédé de la revendication 1, dans lequel les cellules NKT sont collectées à partir d'un tissu pancréatique.

4. Procédé de la revendication 1, dans lequel les cellules NKT sont collectées à partir d'un tissu sanguin.

5. Procédé de la revendication 1, dans lequel le niveau d'expression du marqueur dans l'échantillon de cellules NKT est différent du niveau normal d'expression du marqueur dans un sujet qui n'est pas atteint du diabète du type I, par un facteur d'au moins 2.

6. Procédé de la revendication 1, dans lequel le niveau d'expression du marqueur dans l'échantillon est différent du niveau normal d'expression du marqueur dans un sujet qui n'est pas atteint du diabète du type I, par un facteur d'au moins 5.

7. Procédé de la revendication 1, dans lequel le niveau d'expression du marqueur dans l'échantillon est évalué en détectant la présence dans l'échantillon d'une protéine correspondant au marqueur.

8. Procédé de la revendication 7, dans lequel la présence de la protéine est détectée en utilisant un réactif qui est lié spécifiquement à la protéine.

9. Procédé de la revendication 8, dans lequel le réactif est sélectionné à partir du groupe consistant en un anticorps, un dérivé d'un anticorps, et un fragment d'un anticorps.

10. Procédé de la revendication 1, dans lequel le niveau d'expression du marqueur dans l'échantillon est évalué en détectant la présence dans l'échantillon d'un polynucléotide transcrit ou une partie de celui-ci, où le polynucléotide transcrit comprend le marqueur.

11. Procédé de la revendication 10, dans lequel le polynucléotide transcrit est un ARN messager.

12. Procédé de la revendication 10, dans lequel le polynucléotide transcrit est un ADN complémentaire.

13. Procédé de la revendication 10, dans lequel l'étape de détection comprend en plus une amplification du polynucléotide transcrit.

14. Procédé de la revendication 1, dans lequel le niveau d'expression du marqueur dans l'échantillon est évalué en détectant la présence dans l'échantillon d'un polynucléotide transcrit qui est recuit avec le marqueur ou est recuit avec une partie d'un polynucléotide, où le polynucléotide comprend le marqueur, sous des conditions d'hybridation stringentes.

15. Procédé de la revendication 1, comprenant le fait de comparer:
a) le niveau d'expression dans l'échantillon de cellules NKT de chacun d'une pluralité de marqueurs sélectionnés indépendamment du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B, et
b) le niveau normal d'expression de chacun de la pluralité de marqueurs dans des échantillons du même type obtenus à partir de sujets de contrôle qui ne sont pas atteints du diabète du type I, où le niveau d'expression de plus d'un marqueur parmi les marqueurs est altéré de manière significative, par rapport aux niveaux d'expression normaux correspondants des marqueurs, indiquant que le sujet est atteint du diabète du type I.

16. Procédé de la revendication 15, dans lequel la pluralité comprend deux ou plusieurs des marqueurs.

17. Procédé de la revendication 15, dans lequel la pluralité comprend au moins cinq des marqueurs.

18. Procédé pour surveiller la progression du diabète du type I dans un sujet, le procédé comprenant:
a) détecter dans un échantillon du sujet de cellules NKT à un premier point temporel, l'expression d'un marqueur, où le marqueur est sélectionné du groupe constitué de:
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B, et des combinaison de ceux-ci;
b) répéter l'étape a) à un point temporel subséquent; et c) comparer le niveau d'expression détecté dans les étapes a) et b), et d'après cette comparaison, surveiller la progression du diabète du type I au sein du sujet.

19. Procédé de la revendication 18, dans lequel le marqueur correspond à un polynucléotide transcrit ou à une partie de celui-ci, où le polynucléotide comprend le marqueur.

20. Procédé de la revendication 18, dans lequel les cellules NKT sont collectées à partir d'un tissu pancréatique.

21. Procédé de la revendication 18, dans lequel les cellules NKT sont collectées à partir d'un tissu sanguin.

22. Procédé pour évaluer l'efficacité d'une thérapie pour empêcher le diabète du type I, le procédé comprenant le fait de comparer:
a) une expression d'un marqueur dans un premier échantillon de cellules NKT obtenues à partir du sujet avant de fournir au moins une partie de la thérapie au sujet, où le marqueur est sélectionné à partir du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B, et
b) une expression du marqueur dans un deuxième échantillon de cellules NKT obtenues à partir du sujet suite à la fourniture de la partie de la thérapie, où un niveau d'expression du marqueur qui est abaissé de manière significative dans le deuxième échantillon, par rapport au premier échantillon, est une indication que la thérapie est efficace pour empêcher le diabète du type I dans le sujet.

23. Procédé pour évaluer l'efficacité d'un composé d'essai pour empêcher le diabète du type I dans un sujet, le procédé comprenant le fait de comparer:
a) une expression d'un marqueur dans un premier échantillon de cellules NKT obtenues à partir du sujet et exposées à ou maintenues en présence du composé d'essai, où le marqueur est sélectionné à partir du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, Obi-1, LPAP, Calponine, GRK-6, TXK, homologue de la protéine BTF-3, EIF-4B, et
b) une expression du marqueur dans un deuxième échantillon de cellules NKT obtenues à partir du sujet , où le deuxième échantillon n'est pas exposé au composé d'essai, où un niveau d'expression du marqueur qui est inférieur de manière significative dans le premier échantillon, par rapport au deuxième échantillon, est une indication que le composé d'essai est efficace pour empêcher le diabète du type I dans le sujet.

24. Procédé de la revendication 23, dans lequel les premier et deuxième échantillons sont des parties d'un seul échantillon de cellules NKT obtenues à partir du sujet.

25. Procédé de la revendication 23, dans lequel les premier et deuxième échantillons sont des parties d'échantillons groupés de cellules NKT obtenues à partir du sujet.

26. Procédé pour sélectionner une composition pour empêcher le diabète du type I dans un sujet, le procédé comprenant:
a) maintenir séparément des aliquotes d'un échantillon comprenant des cellules NKT obtenues à partir d'un sujet en présence d'une pluralité de compositions d'essai;
b) comparer une expression d'un marqueur dans chacune des aliquotes, où le marqueur est sélectionné à partir du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B, et
c) sélectionner l'une des compositions d'essai qui cause un niveau inférieur d'expression du marqueur dans l'aliquote contenant cette composition d'essai, par rapport aux autres compositions d'essai.

27. Procédé pour évaluer le potentiel d'un composé d'essai pour déclencher le diabète du type I dans une cellule, le procédé comprenant:
a) maintenir des aliquotes séparées de cellules NKT en présence et à défaut du composé d'essai; et
b) comparer une expression d'un marqueur dans chacune des aliquotes, où le marqueur est sélectionné partir du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B,
où un niveau d'expression du marqueur amélioré de manière significative dans l'aliquote maintenue en présence du composé d'essai, par rapport à l'aliquote maintenue à défaut du composé d'essai, est une indication que le composé d'essai possède le potentiel pour déclencher le diabète du type I dans une cellule.

28. Utilisation d'une trousse pour diriger les procédés selon l'une quelconque des revendications 1, 23, 26 et 27, où la trousse comprend au moins un réactif pour évaluer l'expression d'un marqueur dans un échantillon de cellules NKT, où le marqueur est sélectionné à partir du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B.

29. Utilisation selon la revendication 28, dans lequel le réactif est une sonde d'acide nucléique qui est liée spécifiquement à un polynucléotide transcrit correspondant à un marqueur sélectionné à partir du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, Obi-1, LPAP, Calponine, GRK-6, TXK, homologue de la protéine BTF-3, EIF-4B.

30. Utilisation selon la revendication 28, dans lequel le réactif est un anticorps qui est lié spécifiquement à une protéine correspondant à un marqueur sélectionné du groupe constitué de
LT-β, Sémaphorine III, protéine Frizzled, homologue de la protéine Flightless I., protéine nucléaire pp32, PTP D1, RNP B5, homologue de la protéine RNP, TRAP-3, MLN62, IL-9R, CD37, EBI-1, LPAP, Calponine, GRK-6, TXK, homologue de la protéine BTF-3, EIF-4B.
